# EUROPEAN PATENT SPECIFICATION

(11) **EP 4 025 691 B1**
(45) Date of publication and mention of the grant of the patent: **13.09.2023**
(21) Application number: 21765836.8
(22) Date of filing: 20.07.2021
(51) Int. Cl.: C12N 9/22, C12N 15/10, C12N 15/82

(54) **NOVEL, NON-NATURALLY OCCURRING CRISPR-CAS NUCLEASES FOR GENOME EDITING**
NEUE, NICHT NATÜRLICH VORKOMMENDE CRISPR-CAS-NUKLEASEN ZUR GENOMEDITIERUNG
NOUVELLES NUCLÉASES CRISPR-CAS NON NATURELLES POUR L'ÉDITION DES GÉNOMES

(30) Priority: 21.07.2020 EP 20000262; 10.09.2020 EP 20000329
(43) Date of publication of application: 13.07.2022
(73) Proprietor: BRAIN Biotech AG, 64673 Zwingenberg (DE)
(72) Inventor: SCHOLZ, Paul, 50679 Köln (DE); ZUREK, Christian, 64653 Lorsch (DE); KROHN, Michael, 64653 Lorsch (DE)
(74) Representative: Weickmann & Weickmann PartmbB
(86) International application number: PCT/EP2021/000081
(87) International publication number: WO 2022/017633

(56) References cited:
- WO-A1-2019/030695
- WO-A2-2017/141173
- WO-A2-2018/191715
- Matthew B Begemann ET AL: "Characterization and Validation of a Novel Group of Type V, Class 2 Nucleases for in vivo Genome Editing", bioRxiv, 27 September 2017 (2017-09-27), XP055515734, DOI: 10.1101/192799 Retrieved from the Internet: URL:https://www.biorxiv.org/content/biorxi v/early/2017/09/23/192799.full.pdf

## Description

The present invention is defined by the claims and accordingly relates to a nucleic acid molecule encoding an RNA-guided DNA endonuclease, which is (a) a nucleic acid molecule encoding the RNA-guided DNA endonuclease comprising or consisting of the amino acid sequence of SEQ ID NO: 29, 1 or 3; (b) a nucleic acid molecule comprising or consisting of the nucleotide sequence of SEQ ID NO: 30, 2 or 4; (c) a nucleic acid molecule encoding a RNA-guided DNA endonuclease the amino acid sequence of which is at least 95 % identical to the amino acid sequence of (a); (d) a nucleic acid molecule encoding a RNA-guided DNA endonuclease and comprising or consisting of a nucleotide sequence which is at least 95 % identical to the nucleotide sequence of (b); or (e) the nucleic acid molecule of any one of (a) to (d) wherein T is replaced by U.

CRISPR-Cas systems are widespread adaptive immunity systems of prokaryotes against invading foreign nucleic acids. So far, more than 30 different CRISPR-Cas systems have been identified that differ in their loci architecture, number, and identity of their genes encoding for the Cas (CRISPR-associated) proteins. For example, WO 2018/191715 describes polypeptides with type V CRISPR activity and uses thereof.

The typical signature of the CRISPR systems in prokaryotic genomes is the presence of short (30-45 bp) repetitive sequences (repeats) that are intervened by variable sequences (spacers) of similar lengths. The Cas proteins are located either upstream or downstream of the repeat-spacer cluster. According to their gene composition and mechanistic differences, the subtypes are classified into two CRISPR classes (Class 1 and 2). One of their major differences is that Class 1 CRISPR systems need a complex of multiple Cas proteins to degrade DNA, whereas Class 2 Cas proteins are single, large multidomain nucleases. The sequence-specificity of the Class 2 Cas proteins can simply be modified by synthetic CRISPR RNAs (crRNAs) in order to introduce targeted double-stranded DNA breaks. Most prominent members of such Class 2 Cas proteins are Cas9, Cpf1 (Cas12a) and Cms1, which are harnessed for genome-editing and successfully applied in many eukaryotic organisms including fungi, plants and mammalian cells. Whereas Cas9 and its orthologs are Class2 type II CRISPR nucleases, Cpf1 (WO2016/205711 BROAD Inst.; WO2017/141173 Benson Hill) and Cms1 (WO2019/030695 Benson Hill) belong to Class 2 type V nucleases. Cms1 and Cpf1 CRISPR nucleases are a class of CRISPR nucleases that have certain desirable properties compared to other CRISPR nucleases such as type II nucleases. For instance, in contrast to Cas9 nucleases Cms1 and Cpf1 do not require a trans-activating crRNA (tracrRNA), which is partially complementary to the precursor crRNA (pre-crRNA) (Deltcheva et al. (2011), Nature, 471(7340):602-607). Base-pairing of tracrRNA and pre-crRNA forms a Cas9-bound RNA:RNA duplex, which gets processed by RNase III and other unidentified nucleases. This mature tracrRNA:crRNA duplex mediates the target DNA recognition and cleavage by Cas9. In contrast, type V nucleases can process the pre-crRNA without the need for tracrRNA or cellular nucleases (like RNase III), which significantly simplifies the application of type V nucleases for (multiplex) genome editing.

Several new Class 2 proteins, like C2c1 (Cas12b), C2c2 (Cas13a) and C2c3 (Cas12c) have been identified in the genomes of cultivated bacteria or public available metagenomics datasets, e.g. gut metagenome (Shmakov et al. (2015), Mol Cell,60(3):385-97). According to the recent classification of CRISPR-Cas systems, Class 2 comprises 3 types and 17 subtypes (Makarova et al. (2020), Nat Rev Microbiol, 18(2):67-83).

Moreover, in a recent publication two new Class 2 proteins were discovered (CasX (Cas12a) and CasY (Cas12d)) in uncultivated prokaryotes by metagenome sequencing (Burstein et al. (2017), Nature, 542:237-241), indicating the presence of untapped Cas proteins from organisms which are not cultivated and/or identified yet.

As discussed, the known CRISPR-Cas systems display certain differences as regards their mode of action. These molecular differences not only enlarge the possibilities of using the CRISPR-Cas system for genome editing in a broad range of different genetic backgrounds but also to circumvent issues of particular Cas nucleases when applied in certain organisms, e.g. pre-existing immune response to Cas9 in humans (Charlesworth et al. (2019), Nat Med, 25(2):249-254). Therefore, the identification of Cas nucleases from bacterial species with less direct contact to higher eukaryotes or with a non-native origin is of particular importance. It can be assumed that CRISPR-Cas systems with yet unknown characteristics exist in nature or can be designed by protein engineering. Hence, although already several different CRISPR-Cas systems are known from the prior art there is an ongoing need to identify further RNA-guided DNA endonucleases.

Accordingly, the present invention relates in first aspect to a nucleic acid molecule encoding an RNA-guided DNA endonuclease, which is (a) a nucleic acid molecule encoding the RNA-guided DNA endonuclease comprising or consisting of the amino acid sequence of SEQ ID NO: 29, 1 or 3; (b) a nucleic acid molecule comprising or consisting of the nucleotide sequence of SEQ ID NO: 30, 2 or 4; (c) a nucleic acid molecule encoding a RNA-guided DNA endonuclease the amino acid sequence of which is at least 95 % identical to the amino acid sequence of (a); (d) a nucleic acid molecule encoding a RNA-guided DNA endonuclease and comprising or consisting of a nucleotide sequence which is at least 95 % identical to the nucleotide sequence of (b); or (e) the nucleic acid molecule of any one of (a) to (d) wherein T is replaced by U.

SEQ ID NOs 1, 3 and 29 are the amino acid sequences of the novel CRISPR-Cas endonucleases BEC85, BEC67 and BEC10, respectively, wherein BEC is an abbreviation of BRAIN Engineered Cas. Among the amino acid sequences of SEQ ID NOs 1, 3 and 29 SEQ ID NO: 29 and, thus, the amino acid sequence of BEC10 is preferred. The novel CRISPR-Cas endonucleases BEC85, BEC67 and BEC10 are encoded by the nucleotide sequence of SEQ ID NO: 2, 4 and 30, respectively. Among the nucleotide sequences of SEQ ID NOs 2, 4 and 30 SEQ ID NO: 30 and, thus, the nucleotide sequence of BEC10 is preferred. As will be discussed in more detail herein below, the novel CRISPR-Cas endonucleases BEC85, BEC67 and BEC10 do not occur in nature but have been prepared by protein engineering.

In accordance with the present invention the term "nucleic acid molecule" defines a linear molecular chain of nucleotides. The nucleic acid molecules according to the present invention consist of at least 3327 nucleotides. The group of molecules designated herein as "nucleic acid molecules" also comprises complete genes. The term "nucleic acid molecule" is interchangeably used herein with the term "polynucleotide".

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases. Included are also single- and double-stranded hybrid molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphorarnidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'- and 3'- non-coding regions, and the like.

The term "polypeptide" as used herein interchangeably with the term "protein" describes linear molecular chains of amino acids, including single chain proteins or their fragments. The polypeptides / proteins according to the present invention contain at least 1108 amino acids. Polypeptides may further form oligomers consisting of at least two identical or different molecules. The corresponding higher order structures of such multimers are, correspondingly, termed homo- or heterodimers, homo- or heterotrimers etc. The polypeptides of the invention may form heteromultimers or homomultimers, such as heterodimers or homodimers. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" also refer to naturally modified polypeptides and proteins where the modification is affected e.g. by glycosylation, acetylation, phosphorylation, ubiquitinylation and similar modifications which are well known in the art.

The term "RNA-guided DNA endonuclease" or "CRISPR(-Cas) endonuclease" describes an enzyme having the capability of cleaving the phosphodiester bond within a deoxyribonucleotide (DNA) strand thereby producing a double-strand break (DSB). BEC85, BEC67 and BEC10 are classified as novel type V class 2 CRISPR nucleases which are known to introduce a staggered cut with a 5' overhang. Hence, an RNA-guided DNA endonuclease comprises an endonuclease domain, in particular a RuvC domain. The RuvC domains of BEC85, BEC67 and BEC10 each comprise three split RuvC motifs (RuvC I-III; SEQ ID NO: 5 to 7). A RNA-guided DNA endonuclease also comprises a domain being capable of binding to a crRNA, also known as guide RNA (gRNA; also being designated DNA-targeting RNA herein).

The cleavage site of the RNA-guided DNA endonuclease is guided by a guide RNA. The gRNA confers the target sequence specificity to the RNA-guided DNA endonuclease. Such gRNAs are non-coding short RNA sequences which binds to the complementary target DNA sequences. The gRNA first binds to the RNA-guided DNA endonuclease by a binding domain that can interact with the RNA-guided DNA endonuclease. The binding domain that can interact with the RNA-guided DNA endonuclease typically comprises a region with a stem-loop structure. This stem-loop preferably comprises the sequence UCUACN₃₋₅GUAGAU (SEQ ID NO: 8), with "UCUAC" and "GUAGA" base-pairing to form the stem of the stem-loop. N₃₋₅ denotes that any base may be present at this location, and 3, 4, or 5 nucleotides may be included at this location. The stem-loop most preferably comprises the stem loop direct repeat sequence of BEC85 (SEQ ID NO: 9), BEC67 (SEQ ID NO: 10) and BEC10 (SEQ ID NO: 10), respectively, but in the form of RNA (i.e. wherein T is replaced by U). The gRNA sequence guides the complex (known as CRISPR ribonucleoprotein (RNP) complex of the gRNA and the RNA-guided DNA endonuclease) via pairing to a specific location on a DNA strand, where the RNA-guided DNA endonuclease performs its endonuclease activity by cutting the DNA strand at the target site. The genomic target site of the gRNA can be any about 20 (typically 17 to 26) nucleotide DNA sequence, provided it meets two conditions: (i) The sequence is unique compared to the rest of the genome, and (ii) the target is present immediately adjacent to a Protospacer Adjacent Motif (PAM).

The cleavage site of the RNA-guided DNA endonuclease is, thus, furthermore defined by a PAM. The PAM is a short DNA sequence (usually 2-6 base pairs in length) that follows the DNA region targeted for cleavage by the CRISPR system. The exact sequence depends on which CRISPR endonuclease is used. CRISPR endonucleases and their respective PAM sequences are known in the art (see https://www.addgene.org/crispr/guide/#pam-table). For instance, the PAM being recognized by the first identified RNA-guided DNA endonuclease Cas9 is 5'-NGG-3' (where "N" can be any nucleotide base). The PAM is required for a RNA-guided DNA endonuclease to cut. In Cas9 it is found about 2-6 nucleotides downstream of the DNA sequence targeted by the guide RNA and 3-6 nucleotides downstream from the cut site. In type V systems (including BEC85, BEC67 and BEC10) the PAM is located upstream of both, the target sequence and the cleavage site. The complex of the RNA-guided DNA endonuclease and the guide RNA comprises a so-called PAM interacting domain (Andres et al. (2014), Nature, 513(7519):569-573). Hence, the genomic locations that can be targeted for editing by an RNA-guided DNA endonuclease are limited by the presence and locations of the nuclease-specific PAM sequence. As BEC85, BEC67 and BEC10 belong to the group of Type V Class 2 CRISPR nucleases a T rich PAM site is predicted and a TTTA PAM site was shown to be functional (see examples).

The term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 70 % identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Amino acid sequence as well as nucleotide sequence analysis and alignments in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402). The skilled person is aware of additional suitable programs to align nucleic acid sequences.

As defined herein above, an amino acid sequence and nucleotide sequence identity of at least 95 % is envisaged by the invention. Furthermore, are envisaged with increasing preference at least 96 %, at least 97 %, at least 98 %, at least 99 %, at least 99.5 %, at least 99.8 %, and at least 99.9 % identity by the invention.

With respect to these amino acid sequences and the amino acid sequences being encoded by these nucleotide sequences it is preferred that they maintain or essentially maintain the RNA-guided DNA endonuclease activity of SEQ ID NO: 1, 3 and 29 of the invention. Hence, what is maintained or essentially maintained is the capability to bind to the gRNA to form a complex being capable of binding to the DNA target site of interest, where the endonuclease activity induces a DSB.

The maintenance or essentially maintenance of the RNA-guided DNA endonuclease activity can be analysed in a CRISPR-Cas genome editing experiment, for example, as is illustrated in Example 3-5. It is preferred that the amino acid sequences comprise and the nucleotide sequences encode a RuvC domain as shown in SEQ ID NO: 5 to 7. As mentioned, the RuvC domain is an endonuclease domain.

As can be taken from the appended examples the novel CRISPR nucleases BEC85, BEC67 and BEC10 of the present invention have been generated using protein engineering and an *in silica* based approach. Hence, the Cas nucleases of the present invention were not simply isolated from a bacterial species but are of non-native origin. In more detail, a screening of numerous engineered nuclease sequences was conducted and the activities of the identified sequences were optimized using protein engineering. To the best knowledge of the inventors, this is the first time that a novel type of Cas nuclease has been developed which is not directly related to a sequence found in nature.

Moreover, the experimental results with the novel CRISPR nucleases BEC85, BEC67 and BEC10 in the appended examples of the present application surprisingly showed a different molecular mechanism of CRISPR nucleases of the BEC family in comparison to classical CRISPR Cas nucleases. For instance, in comparison to the Cas9 nuclease, which assists homologous recombination by introducing a RNA directed double-strand break, BEC85, BEC67 and BEC10 mediated editing leads to a strong overall clone reduction in connection with a significant enrichment of cells that successfully accomplished homologous recombination. For this reason, the novel BEC type CRISPR nucleases further enlarge the possibility to use the CRISPR technology for efficient genome editing.

As a proof of principle Example 3 shows that BEC85, BEC67 and BEC10 are active CRISPR-Cas endonucleases that can be successfully used for genome editing. In Example 3 the Ade2 gene of *Saccharomyces cerevisiae* was knocked out using BEC85, BEC67 or BEC10, a gRNA and a homology directed repair template.

Just as the type V CRISPR endonucleases Cms1 and Cpf1, BEC85, BEC67 and BEC10 do not require a trans-activating crRNA (tracrRNA). Moreover, the BEC85, BEC67 and BEC10 containing CRISPR system identified in the present application contain CRISPR repeat sequences with an RNA stem loop at 3'-end of the repeat that is conserved in crRNAs of Cpf1 and Cms1 protein families and the "nearest neighbours" of BEC85, BEC67 and BEC10 among all known CRISPR-Cas endonucleases are CMS like Cas proteins from WO 2017/141173 and in particular the CMS like Cas proteins SuCms1 (Begemann et al. (2017), bioRxiv) and SeqID63 (WO 2019/030695). Interestingly, the activity profile of CMS CRISPR nucleases described in WO 2017/141173, WO 2019/030695 and Begemann et al. (2017), bioRxiv is completely different compared to the activity profile of CRISPR nucleases of the BEC family. Example 3 shows that the endonuclease activity of BEC85, BEC67 and BEC10 is based on a novel molecular mechanism which has not been described before. In more detail, in Example 3 results with the prior art CRISPR endonuclease SpCas9 and the novel CRISPR endonuclease of the invention BEC85, BEC67 and BEC10 are provided. The results surprisingly revealed a completely different molecular genome editing mechanism of the three BEC-type CRISPR nucleases in comparison to the classical CRISPR Cas nuclease SpCas9. While SpCas9 assists homologous recombination by introducing an RNA directed double strand break, BEC85, BEC67 and BEC10 mediated editing leads to a strong overall clone reduction in connection with a significant enrichment of cells that successfully accomplished homologous recombination. Example 3 proves the capability of BEC-type CRISPR nucleases to function as a novel genome editing tool by site directed, highly efficient homology directed recombination.

For this reason, BEC85, BEC67 and BEC10 can be classified as novel, non-naturally occurring Class 2 type V nucleases with overall no significant sequence identity to the known collection of Class 1 and Class 2 CRISPR-Cas endonucleases and with overall low sequence identity to individual Cms1-type endonucleases.

BEC85, BEC67 and BEC10 are novel CRISPR-Cas endonucleases which are significantly distinct from the known collection of CRISPR-Cas endonucleases and are showing a novel mechanism of activity, BEC85, BEC67 and BEC10 expand the known collection of CRISPR-Cas endonucleases applicable for genome editing, gene regulation and nucleic acid enrichment/purification in different biotechnological and pharmaceutical sectors. The results described in Example 3 strongly indicate that BEC type CRISPR nucleases are not only a novel type of effector proteins with distinct locus architectures but also display a new molecular genome editing mechanism.

Furthermore, Example 4 demonstrates that genome editing using the novel BEC family type nucleases of the invention provide for significantly higher clone reduction numbers and significantly superior editing ratios as comparted to their next neighbor sequences SuCms1 (Begemann et al. (2017), bioRxiv) and SeqID63 (WO 2019/030695). The results in Example 4 further prove the general superiority of the BEC type nucleases for genome editing as compared to the previously known CRISPR Cas nucleases.

Yet further, Example 5 demonstrates that the novel BEC family type nucleases of the invention display strong activity at temperature levels from 21°C to 37°C and in particular a superior genome editing efficiency and colony reduction rate as compared to the next neighbor sequences SuCms1 and SeqID63. For instance, the genome editing efficiency of the SuCms1 nuclease significantly decreases at 21°C to levels comparable to the negative control (0.3 %) whereas the BEC10 editing efficiency remains at a high level (65 %) even at the relative low temperature of 21°C. High activity within a temperature range from 21°C to 37°C is of great interest for biotechnological, agricultural and pharmaceutical applications because within this temperature range various types of cells are cultured (e.g. various plants and plant cells ≈ 21°C, various yeast and fungal cells ≈ 30°C, various prokaryotic organisms and mammalian cell lines ≈ 37°C). The novel BEC family type nucleases therefore advantageously allow the design of universally applicable CRISPR systems.

In accordance with a preferred embodiment of the first aspect of the invention the nucleic acid molecule is operably linked to a promoter that is native or heterologous to the nucleic acid molecule.

A promoter is a region of DNA that leads to initiation of transcription of a particular gene. Promoters are generally located near the transcription start sites of genes, upstream on the DNA (towards the 5' region of the sense strand). Promoters are typically 100-1000 base pairs long. For transcription to take place, the enzyme that synthesizes RNA, known as RNA polymerase, must attach to the DNA near a gene. Promoters contain specific DNA sequences such as response elements that provide a secure initial binding site for RNA polymerase and for proteins called transcription factors that recruit RNA polymerase. Hence, the binding of the RNA polymerase and transcription factors to the promoter site ensures the transcription of the gene.

In this connection the term "operably linked" defines that the promoter is linked to the gene of the same DNA strand, such that upon binding of the RNA polymerase and transcription factors the transcription of the gene is initiated. Generally, each gene is operably linked in its natural environment of the genome of a living organism to a promoter. This promoter is designated "natural promoter" or "wild-type promoter" herein. A heterologous promoter is distinct from the natural promoter or wild-type promoter. Hence, a nucleic acid molecule which is operably linked to a promoter that is heterologous to the nucleic acid molecule does not occur in nature.

Heterologous promoters that can be used to express a desired gene are known in the art and can, for example, be obtained from the EPD (eukaryotic promoter database) or EDPnew (https://epd.epfl.ch//index.php). In this database eukaryotic promoters including animal, plant and yeast promoters can be found.

The promoter can, for example, be a constitutively active, inducible, tissue-specific, or developmental stage-specific promoter. By using such promoter the desired timing and site of expression can be regulated.

The *AOX1* or *GAL1* promoter in yeast or the CMV- (Cytomegalovirus), SV40-, RSV-promoter (Rous sarcoma virus), chicken beta-actin promoters, CAG-promoter (a combination of chicken beta-actin promoter and cytomegalovirus immediate-early enhancer), the gai10 promoter, human elongation factor 1α-promoter, CaM-kinase promoter, and the Autographa californica multiple nuclear polyhedrosis virus (AcMNPV) polyhedral promoter are examples of constitutively active promoters.

Examples of inducible promoters are the Adhl promoter which is inducible by hypoxia or cold stress, the Hsp70 promoter which is inducible by heat stress, the PPDK promoter and the pepcarboxylase promoter which are both inducible by light. Also useful are promoters which are chemically inducible, such as the In2-2 promoter which is safener induced (US 5,364,780), the ERE promoter which is estrogen induced, and the Axigl promoter which is auxin induced and tapetum specific but also active in callus (WO03060123).

A tissue-specific promoter is a promoter that initiates transcription only in certain tissues. A developmental stage-specific promoter is a promoter that initiates transcription only in a certain developmental stage.

In the examples herein below a Tpi1 (SEQ ID NO: 12) and a SNR52 promoter (SEQ ID NO: 18) have been used. Therefore, the use of a Tpi1 and a SNR52 promoter are preferred.

In accordance with a further preferred embodiment of the first aspect of the invention the nucleic acid molecule is linked to a nucleic acid sequence encoding a nuclear localization signal (NLS).

Further details on NLS will be provided herein below.

In accordance with another preferred embodiment of the first aspect of the invention said nucleic acid molecule is codon-optimized for expression in a eukaryotic cell, preferably a yeast, plant or animal cell.

As discussed, BEC85, BEC67 and BEC10 were generated by protein engineering and are non-naturally occurring CRISPR nucleases.

The genes encoding the BEC85, BEC67 and BEC10 polypeptides, can be codon-optimized for expression in the target cell, and can optionally include a sequence encoding an NLS and/or a peptide tag, such a purification tag. Further details on the tag will be provided herein below.

Codon optimization is a process used to improve gene expression and increase the translational efficiency of a gene of interest by accommodating codon bias of the host cell. A "codon-optimized gene" is therefore a gene having its frequency of codon usage designed to mimic the frequency of preferred codon usage of the host cell. Nucleic acid molecules can be codon optimized, either wholly or in part. Because any one amino acid (except for methionine and tryptophan) is encoded by a number of codons, the sequence of the nucleic acid molecule may be changed without changing the encoded amino acid. Codon optimization is when one or more codons are altered at the nucleic acid level such that the amino acids are not changed but expression in a particular host organism is increased. Those having ordinary skill in the art will recognize that codon tables and other references providing preference information for a wide range of organisms are available in the art (see, e.g., Zhang et al. (1991) Gene 105:61-72; Murray et al. (1989) Nucl. Acids Res. 17:477-508). Methodology for optimizing a nucleotide sequence for expression is provided, for example, in U.S. Pat. No. 6,015,891. Programs for codon optimization are available in the art (e.g., OPTIMIZER at genomes.urv.es/OPTIMIZER; OptimumGene.TM. from GenScript at: www.genscript.com/codon_opt.html).

The eukaryotic cell is preferably a yeast cell and accordingly the codon-optimization is preferably an optimization for the expression in yeast cells. Yeast cells are of particular commercial interest since they are one of the most commonly used eukaryotic hosts for the industrial production of recombinant proteins.

In another embodiment the eukaryotic cell is a mammalian cell and accordingly the codon-optimization is preferably an optimization for the expression in mammalian cells. Also mammalian cells, preferably CHO and HEK293 cells, are of particular commercial interest since they are commonly used hosts for the industrial production of recombinant protein therapeutics.

Further details on suitable eukaryotic cells, including plant and animal cells will be provided herein below.

In Example 2, BEC85, BEC67 or BEC10 encoding nucleotide sequences are described being codon-optimized for the expression in yeast (in particular *Saccharomyces cerevisiae*) or bacteria (*E. coli*)

The present invention relates in a second aspect to a vector encoding the nucleic acid molecule of the first aspect.

The definitions and preferred embodiments as described herein above apply *mutatis mutandis* to the second aspect, if applicable.

A vector according to this invention is generally and preferably capable of directing the replication, and/or the expression of the nucleic acid molecule of the invention and/or the expression of the polypeptide encoded thereby.

Preferably, the vector is a plasmid, cosmid, virus, bacteriophage or another vector used conventionally e.g. in genetic engineering.

Exemplary plasmids and vectors are listed, for example, in Studier and coworkers (Studier, W.F.; Rosenberg A.H.; Dunn J.J.; Dubendroff J.W., 1990, Use of the T7 RNA polymerase to direct expression of cloned genes, Methods Enzymol. 185, 61-89) or the brochures supplied by the companies Novagen, Promega, New England Biolabs, Clontech and Gibco BRL. Other preferred plasmids and vectors can be found in: Glover, D.M., 1985, DNA cloning: a practical approach, Vol. I-III, IRL Press Ltd., Oxford; Rodriguez, R.L. and Denhardt, D.T. (eds), 1988, Vectors: a survey of molecular cloning vectors and their uses, 179-204, Butterworth, Stoneham; Goedeel, D.V., 1990, Systems for heterologous gene expression, Methods Enzymol. 185, 3-7; Sambrook, J.; Russell, D. W., 2001, Molecular cloning: a laboratory manual, 3rd ed., Cold Spring Harbor Laboratory Press, New York.

Particularly preferred vectors are vectors that can be used for CRISPR genome editing, in particular vectors only expressing the nucleic acid molecule of the invention encoding the RNA-guided DNA endonuclease or vectors expressing both, the nucleic acid molecule of the invention encoding the RNA-guided DNA endonuclease and the guide RNA (so called "all-in one vectors"). In the former case a second vector is to be employed for the expression of the guide RNA. CRISPR genome editing vectors are commercially available, for example, from OriGene, Vector Builder or ThermoFisher.

The nucleic acid molecule of the present invention referred to above may also be inserted into vectors such that a translational fusion with another nucleic acid molecule is generated. To this aim, overlap extension PCR can be applied (e.g. Wurch, T., Lestienne, F., and Pauwels, P.J., A modified overlap extension PCR method to create chimeric genes in the absence of restriction enzymes, Biotechn. Techn. 12, 9, Sept. 1998, 653-657). The products arising therefrom are termed fusion proteins and will be described further below. The other nucleic acid molecules may encode a protein which may e.g. increase the solubility and/or facilitate the purification of the protein encoded by the nucleic acid molecule of the invention. Non-limiting examples include pET32, pET41, pET43. The vectors may also contain an additional expressible nucleic acid coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta^{®}.

For vector modification techniques, see J.F. Sambrook and D.W. Russell, ed., Cold Spring Harbor Laboratory Press, 2001, ISBN-10 0-87969-577-3. Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M13 origins of replication.

The coding sequences inserted in the vector can e.g. be synthesized by standard methods or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, transcriptional termination sequences, promoters, enhancers, and/or insulators), internal ribosomal entry sites (IRES) (Owens et al., (2001), PNAS. 98 (4) 1471-1476) and optionally poly-A signals ensuring termination of transcription and stabilization of the transcript. Additional regulatory elements may include transcriptional as well as translational enhancers, and/or naturally associated or heterologous promoter regions. The regulatory elements may be native to the endonuclease of the invention or heterologous regulatory elements. Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the nucleic acid molecule of the invention. Such leader sequences are well known in the art. Specifically designed vectors allow the shuttling of DNA between different hosts, such as bacteria-fungal cells or bacteria-animal cells.

Additionally, baculoviral systems or systems based on Vaccinia Virus or Semliki Forest Virus can be used as vectors in eukaryotic expression systems for the nucleic acid molecules of the invention. Expression vectors derived from viruses such as retroviruses, vaccinia virus, adeno-associated virus, herpes viruses, or bovine papilloma virus, may be used for delivery of the nucleic acids or vector into targeted cell populations. Methods which are well known to those skilled in the art can be used to construct recombinant viral vectors; see, for example, the techniques described in Sambrook and D.W. Russell, ed., Cold Spring Harbor Laboratory Press, 2001.

Examples for regulatory elements permitting expression in eukaryotic host cells are promoters, including the promoters as described herein above. Besides elements which are responsible for the initiation of transcription such regulatory elements may also comprise transcription termination signals, such as the SV40-poly-A site or the tk-poly-A site or the SV40, lacZ and AcMNPV polyhedral polyadenylation signals, downstream of the nucleic acid.

The co-transfection with a selectable marker such as kanamycin or ampicillin resistance genes for culturing in *E. coli* and other bacteria allows the identification and isolation of the transfected cells. Selectable markers for mammalian cell culture are the dhfr, gpt, neomycin, hygromycin resistance genes. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS). Using such markers, the cells are grown in selective medium and the cells with the highest resistance are selected.

However, the nucleic acid molecules of the invention as described herein above may also be designed for direct introduction or for introduction via liposomes, phage vectors or viral vectors (e.g. adenoviral or retroviral) into the cell.

The present invention relates in a third aspect to a host cell comprising the nucleic acid molecule of the first aspect or being transformed, transduced or transfected with the vector of the second aspect.

The definitions and preferred embodiments as described herein above apply *mutatis mutandis* to the third aspect, if applicable.

Large amounts of the RNA-guided DNA endonuclease may be produced by said host cell, wherein the isolated nucleotide sequence encoding the RNA-guided DNA endonuclease is inserted into an appropriate vector or expression vector before insertion into the host. The vector or expression vector is introduced into an appropriate host cell, which preferably can be grown in large quantities, and the RNA-guided DNA endonuclease is purified from the host cells or the culture media.

The host cells may also be used to supply the RNA-guided DNA endonuclease of the invention without requiring purification of the RNA-guided DNA endonuclease (see Yuan, Y.; Wang, S.; Song, Z.; and Gao, R., Immobilization of an L-aminoacylase-producing strain of Aspergillus oryzae into gelatin pellets and its application in the resolution of D,L-methionine, Biotechnol Appl. Biochem. (2002). 35:107-113). The RNA-guided DNA endonuclease of the invention may be secreted by host cells. Those skilled in the field of molecular biology will understand that any of a wide variety of expression systems may be used to provide the RNA-guided DNA endonuclease. The precise host cell used is not critical to the invention, so long as the host cells produce the RNA-guided DNA endonuclease when grown under suitable growth conditions.

Host cells into which vectors containing the nucleic acid molecule of the invention can be cloned are used for replicating and isolating a sufficient quantity of the recombinant enzyme. The methods used for this purpose are well known to the skilled person (Sambrook and D.W. Russell, ed., Cold Spring Harbor Laboratory Press, 2001).

The expression of the RNA-guided DNA endonuclease may not only be used to produce the RNA-guided DNA endonuclease in a host cell, but its expression may also be used to edit the genome of the host cell. In such a case the host cell also comprises a guide RNA. Vectors that can be used for CRISPR genome editing have been discussed herein above.

In accordance with a preferred aspect of the third aspect of the invention the host cell is a eukaryotic cell or a prokaryotic cell and is preferably a plant, yeast or an animal cell.

The host cell can be a eukaryotic cell, and can be, for example, the cell of a fungus, algae, plant, or animal, wherein the animal can be an avian, reptile, amphibian, fish, cephalopod, crustacean, insect, arachnid, marsupial, or mammalian. The gene encoding BEC85, BEC67 or BEC10 that is non-native with respect to the host cell can be operably linked to a regulatory element, such as a promoter. The promoter can be native to the host organism or can be a promoter of another species. A construct for expressing BEC85, BEC67 or BEC10 in a heterologous host cell, such as a eukaryotic cell, can optionally further include a transcriptional terminator. The gene encoding BEC85, BEC67 or BEC10 can optionally be codon optimized for the host species, can optionally include one or more introns, and can optionally include one or more peptide tag sequences, one or more nuclear localization sequences (NLSs) and/or one or more linkers or engineered cleavage sites (e.g. a 2a sequence). In various embodiments a host cell can include any of the engineered BEC85, BEC67 or BEC10 CRISPR systems disclosed above, where the nucleic acid sequence encoding the effector is present in the cell prior to introduction of a guide RNA. In other embodiments, the cell that is engineered to include a gene for expressing a BEC85, BEC67 or BEC10 polypeptide can further include a polynucleotide encoding a guide RNA (e.g., a guide RNA) that is operably linked to a regulatory element.

The cell or organism can be a prokaryotic cell. Suitable prokaryotic host cells comprise e.g. bacteria of the species Escherichia, such as strains derived from *E. coli* BL21 (e.g. BL21(DE3), BL21(DE3)PlysS, BL21(DE3)RIL, BL21(DE3)PRARE, BL21 codon plus, BL21(DE3) codon plus), Rosetta^{®}, XL1 Blue, NM522, JM101, JM109, JM105, RR1, DH5α, TOP 10, HB101 or MM294. Further suitable bacterial host cells are, but not limited to, *Streptomyces, Pseudomonas,* such as *Pseudomonas putida, Corynebacterium,* such as C. *glutamicum, Lactobacillus,* such as *L. salivarius, Salmonella,* or *Bacillus* such as *Bacillus subtilis.*

In general, a eukaryotic host cell is preferred over a prokaryotic host cell.

The eukaryotic cell can be a yeast, fungus, amoebae, insect, vertebrate (e.g. mammalian) or plant cells.

Yeasts cells can be, for example, *Saccharomyces cerevisiae, Ogataea angusta, Kluyveromyces sp. such as K. marxianus* or *K. lactis* or *Pichia sp.* such as *P. pastoris, Yarrowia sp.* such as *Yarroawia lipolytica, Candida sp.,* insect cells such as *Drosophila* S2 or *Spodoptera* Sf9 cells, plant cells, or fungi *cells,* preferably of the family *Trichocomaceae,* more preferably of the genus *Aspergillus, Penicillium* or *Trichoderma,* or of the family *Ustilaginaceae,* preferably *Ustilago sp..*

Plant host cells that may be used include monocots and dicots (i.e., monocotyledonous and dicotyledonous, respectively), such as crop plant cells and tobacco cells.

Mammalian host cells that could be used include human Hela, HEK293, H9 and Jurkat cells, mouse NIH3T3 and C127 cells, COS 1, COS 7 and CV1, quail QC1-3 cells, mouse L cells, Bowes melanoma cells, HaCaT cells, BHK, HT29, A431, A549, U2OS, MDCK, HepG2, CaCo-2 and Chinese hamster ovary (CHO) cells.

The present invention relates in a fourth aspect to a plant, seed or a part of a plant, said part of a plant no being a single plant cell, or an animal comprising the nucleic acid molecule of the first aspect or being transformed, transduced or transfected with the vector of the second aspect.

The definitions and preferred embodiments as described herein above apply *mutatis mutandis* to the fourth aspect, if applicable.

The animal is preferably a mammal and most preferably a non-human mammal. The mammal can be, for example, a mouse, rat, hamster, cat, dog, horse, swine, cattle, monkey, ape, etc.

By the expression of the nucleic acid molecule of the first aspect in a plant, seed or a part of a plant or an animal along with a guide RNA the genome of the host may be edited. The genome may be edited, for example, in order to introduce a targeted gene mutation, for gene therapy, for a creating chromosome rearrangement, for studying gene function, for the production of a transgenic organism, for endogenous gene labeling or for targeted transgene addition.

The present invention relates in a fifth aspect to a method of producing an RNA-guided DNA endonuclease comprising culturing the host cell of the third aspect and isolating the RNA-guided DNA endonuclease produced.

The definitions and preferred embodiments as described herein above apply *mutatis mutandis* to the fifth aspect, if applicable.

Suitable conditions for culturing a prokaryotic or eukaryotic host are well known to the person skilled in the art. In general, suitable conditions for culturing bacteria are growing them under aeration in Luria Bertani (LB) medium. To increase the yield and the solubility of the expression product, the medium can be buffered or supplemented with suitable additives known to enhance or facilitate both. *E. coli* can be cultured from 4 to about 37 °C, the exact temperature or sequence of temperatures depends on the molecule to be overexpressed.

In general, *Aspergillus sp.* may be grown on Sabouraud dextrose agar, or potato dextrose agar at about to 10°C to about 40°C, and preferably at about 25°C. Suitable conditions for yeast cultures are known, for example from Guthrie and Fink, "Guide to Yeast Genetics and Molecular Cell Biology" (2002); Academic Press Inc.. The skilled person is also aware of all these conditions and may further adapt these conditions to the needs of a particular host species and the requirements of the polypeptide expressed. In case an inducible promoter controls the nucleic acid of the invention in the vector present in the host cell, expression of the polypeptide can be induced by addition of an appropriate inducing agent. Suitable expression protocols and strategies are known to the skilled person.

Depending on the cell type and its specific requirements, mammalian cell culture can e.g. be carried out in RPMI or DMEM medium containing 10 % (v/v) FCS, 2mM L-glutamine and 100 U/ml penicillin/streptomycin. The cells can be kept at 37°C in a 5 % CO₂, water saturated atmosphere. Suitable expression protocols for eukaryotic cells are well known to the skilled person and can be retrieved e.g. from Sambrook, 2001.

Methods for the isolation of the produced RNA-guided DNA endonuclease are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001.

The step of protein isolation is preferably a step of protein purification. Protein purification in accordance with the invention specifies a process or a series of processes intended to further isolate the polypeptide of the invention from a complex mixture, preferably to homogeneity. Purification steps, for example, exploit differences in protein size, physico-chemical properties and binding affinity. For example, proteins may be purified according to their isoelectric points by running them through a pH graded gel or an ion exchange column. Further, proteins may be separated according to their size or molecular weight via size exclusion chromatography or by SDS-PAGE (sodium dodecyl sulfate-polyacrylamide gel electrophoresis) analysis. In the art, proteins are often purified by using 2D-PAGE and are then further analysed by peptide mass fingerprinting to establish the protein identity. This is useful for scientific purposes and the detection limits for protein are very low and nanogram amounts of protein are sufficient for their analysis. Proteins may also be purified by polarity/hydrophobicity via high performance liquid chromatography or reversed-phase chromatography. Thus, methods for protein purification are well known to the skilled person.

The present invention relates in a sixth aspect to an RNA-guided DNA endonuclease encoded by the nucleic acid molecule of the first aspect.

The definitions and preferred embodiments as described herein above apply *mutatis mutandis* to the sixth aspect, if applicable.

The amino acid sequences of SEQ ID NO: 1, 3 and 29 are particularly preferred examples of the RNA-guided DNA endonuclease of the invention. Most preferred is an RNA-guided DNA endonuclease comprising or consisting of the amino acid sequence of SEQ ID NO: 29.

The RNA-guided DNA endonuclease of the sixth aspect of the invention may also be a fusion protein, wherein the amino acid sequence of the RNA-guided DNA endonuclease is fused to a fusion partner. The fusion may be a direct fusion or a fusion via a linker. The linker is preferably a peptide, such as a GS-linker.

The fusion partner can be located at the N-terminus, the C-terminus, at both termini, or in an internal location of the RNA-guided DNA endonuclease polypeptide, preferably at the N- or C-terminus.

The fusion partner is preferably a nuclear localization signal (NLS), a cell-penetrating domain, a plastid targeting signal, a mitochondrial targeting signal peptide, a signal peptide targeting both plastids and mitochondria, a marker domain, a tag (such as a purification tag), a DNA modifying enzyme or a transactivation domain.

DNA modifying enzymes may modify the DNA by phosphorylation, dephosphorylation of blunting DNA, wherein blunting refers the digestion single-stranded overhangs. Non-limiting examples of dephosphorylation enzymes are the Shrimp Alkaline Phosphatase (rSAP), Quick CIP Phosphatase and Antarctic Phosphatase. Non-limiting examples of phosphorylation enzymes are polynucleotide kinases, such as T4 PNK. Non-limiting examples of blunting enzymes are the DNA Polymerase I Large (Klenow) Fragment, T4 DNA Polymerase or Mung Bean Nuclease.

Transactivation domains (or trans-activating domains (TADs)) are transcription factor scaffold domains which contain binding sites for other proteins such as transcription co-regulators. Non-limiting examples are nine-amino-acid transactivation domains (9aaTADs) and Glutamine (Q)-rich TADs.

In general, an NLS comprises a stretch of basic amino acids. Nuclear localization signals are known in the art. The NLS can be at the N- terminus, the C-terminus or both the RNA-guided DNA endonuclease polypeptide according to the invention. For instance, the RNA-guided DNA endonuclease polypeptide according to the invention may comprise about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g., zero or at least one or more NLS at the amino-terminus and zero or at one or more NLS at the carboxy-terminus). When more than one NLS is present, each may be selected independently of the others, such that a single NLS may be present in more than one copy and/or in combination with one or more other NLSs present in one or more copies. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C- terminus. The RNA-guided DNA endonuclease polypeptide sequence and the NLS may in some embodiments be fused with a linker between 1 to about 20 amino acids in length.

Non-limiting examples of NLSs include an NLS sequence derived from: the NLS of the SV40 virus large T-antigen; the NLS from nucleoplasmin (e.g., the nucleoplasmin bipartite NLS); the c-myc NLS; the hRNPA! M9 NLS; the IBB domain from importin-alpha; the NLS sequences of the myoma T protein, the p53 protein; the c-abl IV protein, or influenza virus NS 1; the NLS of the Hepatitis virus delta antigen, the Mxl protein; the poly(ADP-ribose) polymerase; and the steroid hormone receptors (human) glucocorticoid. In general, the one or more NLSs are of sufficient strength to drive accumulation of the RNA-guided DNA endonuclease polypeptide according to the invention in a detectable amount in the nucleus of a eukaryotic cell.

Plastid, mitochondrial, and dual-targeting signal peptide localization signals are also known in the art (see, e.g., Nassoury and Morse (2005) Biochim Biophys Acta 1743:5-19; Kunze and Berger (2015) Front Physiol 6:259; Herrmann and Neupert (2003) IUBMB Life 55:219-225; Soil (2002) Curr Opin Plant Biol 5:529-535; Carrie and Small (2013) Biochim Biophys Acta 1833:253-259; Carrie et al. (2009) FEBS J 276: 1187-1195; Silva-Filho (2003) Curr Opin Plant Biol 6:589-595; Peeters and Small (2001) Biochim Biophys Acta 1541:54-63; Murcha et al. (2014) Exp Bot 65:6301-6335; Mackenzie (2005) Trends Cell Biol 15:548-554; Glaser et al. (1998) Plant Mol Biol 38:311-338).

Non-limiting examples of marker domains include fluorescent proteins, purification tags, and epitope tags. In certain embodiments, the marker domain can be a fluorescent protein. Non-limiting examples of suitable fluorescent proteins include green fluorescent proteins (e.g., GFP, GFP-2, tagGFP, turboGFP, EGFP, Emerald, Azami Green, Monomeric Azami Green, CopGFP, AceGFP, ZsGreenl), yellow fluorescent proteins (e.g. YFP, EYFP, Citrine, Venus, YPet, PhiYFP, ZsYellowl), blue fluorescent proteins (e.g. EBFP, EBFP2, Azurite, mKalamal, GFPuv, Sapphire, T-sapphire), cyan fluorescent proteins (e.g. ECFP, Cerulean, CyPet, AmCyanl, Midoriishi-Cyan), red fluorescent proteins (mKate, mKate2, mPlum, DsRed monomer, mCherry, mRFPI, DsRed- Express, DsRed2, DsRed-Monomer, HcRed-Tandem, HcRedl, AsRed2, eqFP611, mRasberry, mStrawberry, Jred), and orange fluorescent proteins (mOrange, mKO, Kusabira-Orange).

A tag is a short amino acid sequence that allows the identification of the RNA-guided DNA endonuclease polypeptide according to the invention in a mixture of polypeptides. Hence, the tag is preferably a purification tag. Non-limiting examples of a purification tag are a His-tag (e.g. His-6-tag). a GST-tag, DHFR-tag and a CBP-tag, A review of known purification tags can be found in Kimple et al. (2015), Curr Protoc Protein Sci. 2013; 73: Unit-9.9.

The present invention relates in a seventh aspect to a composition comprising the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof.

The definitions and preferred embodiments as described herein above apply *mutatis mutandis* to the seventh aspect, if applicable.

The term "composition" as used herein refers to a composition comprising at least one of the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof which are also collectively referred in the following as compounds.

In accordance with a preferred embodiment of the seventh aspect the composition is a pharmaceutical composition.

In accordance with the present invention, the term "pharmaceutical composition" relates to a composition for administration to a patient, preferably a human patient. The pharmaceutical composition of the invention comprises at least one of the compounds recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by conventional methods. These pharmaceutical compositions may be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g of the active compound per day. However, a more preferred dosage might be in the range of 0.01 mg to 100 mg, even more preferably 0.01 mg to 50 mg and most preferably 0.01 mg to 10 mg per day. The length of treatment needed to observe changes and the interval following treatment for responses to occur vary depending on the desired effect. The particular amounts may be determined by conventional tests which are well known to the person skilled in the art.

The pharmaceutical composition may be used, for example, to treat or prevent a pathogenic disease, such as a viral or bacterial disease. For instance, the RNA-guided DNA endonuclease of the sixth aspect may be used together with gRNA targeting the genome of the pathogen thereby modifying the genome of the pathogen, such that the disease being caused by the pathogen is prevented or treated.

The pharmaceutical composition may also be used, for example, to treat or prevent a microbiome imbalance. An imbalance in the microbiome can occur, for example, because of an overuse of antibiotics, which may cause an overgrowth of pathogenic bacteria and yeast.

The present invention also relates to a diagnostic composition comprising the RNA-guided DNA endonuclease of claim 11 and a single-stranded DNA strand being attached to a marker, so that when the RNA-guided DNA endonuclease polypeptide cuts the single-stranded DNA, it activates the reporter, causing it to fluoresce or change color.

A "diagnostic composition" relates to composition which is suitable to detect a disease in subject, both infectious and non-infectious disease. The diagnostic composition may in particular comprise a marker portion as described herein above in connection with the fusion protein of the invention being attached to ssDNA strands, so that when RNA-guided DNA endonuclease polypeptide according to the invention cuts the ssDNA, it activates the reporter, causing it to fluoresce or change color, thus enabling visual detection of the specific disease nucleic marker. The diagnostic composition may be applied to a body fluid sample, such as a blood, urine, or saliva.

The present invention relates in a eighth aspect to the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof for use in the treatment of a disease in a subject or a plant by modifying a nucleotide sequence at a target site in the genome of the subject or plant.

The definitions and preferred embodiments as described herein above apply *mutatis mutandis* to the eighth aspect, if applicable.

The modification of a nucleotide sequence at a target site in the genome of the subject or plant is in accordance with the invention a genome editing by the CRISPR technology and in particular by the novel RNA-guided DNA endonucleases as provided herewith which is to be used in combination with a proper gRNA and optionally a repair substrate as described herein below in order to determine the target side of the genome modification.

Genome editing (also known as genome engineering) is a type of genetic engineering in which a target site, preferably a gene of interest is inserted, deleted, modified or replaced in the genome of the cell. The target site, preferably the gene of interest can be in the genome but may also be in the mitochondrial DNA (animal cells) or chloroplast DNA (plant cells). Genome editing may result in a loss-of-function mutation or a gain-of-function mutation in the genome of the cell. A loss-of-function mutation (also called inactivating mutation) results in the gene of interest having less or no function (being partially or wholly inactivated). When the allele has a complete loss of function (wholly inactivated) this is also called herein a (gene) knock-out. A gene knock-out may be achieved by inserting, deleting, modifying or replacing one or more nucleotides of a gene. A gain-of-function mutation (also called activating mutation) may change the gene of interest such that its effect becomes stronger (enhanced activation) or even is superseded by a different (e.g. abnormal) function. A gain-of-function mutation may also introduce a new function or effect into a cell which the cell did not have before. In this context the new gene may be added to the genome of the cell (insertion) or may replace a gene within the genome. A gain-of-function mutation introducing such a new function or effect is also called gene knock-in. Genome editing may also result in the up- or down-regulation of one or more genes. By targeting DNA sites which are responsible for the regulation of the expression of a gene (e.g. a promoter region or a gene encoding transcription factor) the expression of genes can be up- or down-regulated by CRISPR technology. Further details on the mode of action of the CRISPR technology will be provided herein below.

Since its discovery, the CRISPR technology has been increasingly applied to therapeutic genome editing. Employment of several viral and non-viral vectors has enabled efficient delivery of the CRISPR system to target cells or tissues. In addition, the CRISPR system is able to modulate the target gene's expression in various ways, such as mutagenesis, gene integration, epigenome regulation, chromosomal rearrangement, base editing and mRNA editing (for review Le and Kim (2019), Hum Genet.; 138(6):563-590).

The modification of a nucleotide sequence at a target site in the genome of the subject is preferably a gene therapy. Gene therapy is based on the principle of the genetic manipulation of nucleotide sequence at a target site for treating and preventing a disease, in particular a human disease.

In clinical trials of the CRISPR technology, scientists are using the CRISPR technology to combat cancer and blood disorders in humans. In these trials, some cells are removed from the subject to be treated, the DNA is genome-edited and then the genome edited cells are put back into the subject, said cells now being armed to fight the disease to be treated.

The present invention relates in a ninth aspect to an *in vitro* method of modifying a nucleotide sequence at a target site in the genome of a cell comprising introducing into said cell (i) a DNA-targeting RNA or a DNA polynucleotide encoding a DNA-targeting RNA, wherein the DNA-targeting RNA comprises: (a) a first segment comprising a nucleotide sequence that is complementary to a sequence in the target DNA; and (b) a second segment that interacts with the RNA-guided DNA endonuclease of the sixth aspect; and (ii) the RNA-guided DNA endonuclease of the sixth aspect, or the nucleic acid molecule encoding an RNA-guided DNA endonuclease of the first aspect, or the vector of the second aspect, wherein the RNA-guided DNA endonuclease comprises (a) an RNA-binding portion that interacts with the DNA-targeting RNA and (b) an activity portion that exhibits site-directed enzymatic activity.

Accordingly, also described herein is a composition (e.g. a pharmaceutical) comprising (i) a DNA-targeting RNA or a DNA polynucleotide encoding a DNA-targeting RNA, wherein the DNA-targeting RNA comprises: (a) a first segment comprising a nucleotide sequence that is complementary to a sequence in the target DNA; and (b) a second segment that interacts with the RNA-guided DNA endonuclease of the sixth aspect; and (ii) the RNA-guided DNA endonuclease of the sixth aspect, or the nucleic acid molecule encoding an RNA-guided DNA endonuclease of the first aspect, or the vector of the second aspect, wherein the RNA-guided DNA endonuclease comprises (a) an RNA-binding portion that interacts with the DNA-targeting RNA and (b) an activity portion that exhibits site-directed enzymatic activity.

The definitions and preferred embodiments as described herein above apply *mutatis mutandis* to the ninth aspect, if applicable.

The DNA-targeting RNA comprises a first segment comprising a nucleotide sequence that is complementary to a sequence in the target DNA and a second segment that interacts with the RNA-guided DNA endonuclease. As discussed herein above, the nucleotide sequence that is complementary to a sequence in the target DNA defines the target specificity of the RNA-guided DNA endonuclease. As also discussed herein above, the DNA-targeting RNA binds to the RNA-guided DNA endonuclease, whereby a complex is formed. The second segment interacts with the RNA-guided DNA endonuclease and is responsible for the formation of the complex. The second segment that interacts with the RNA-guided DNA endonuclease of the sixth aspect preferably comprises or consists of SEQ ID NO: 8 and more preferably of SEQ ID NO: 9 or 10. SEQ ID NO: 8 is a consensus sequence of the second segment of Type V Class 2 CRISPR nucleases. In Type V Class 2 CRISPR nucleases the second segment is also known as 5' handle. SEQ ID NO: 9 or 10 are the second segments of BEC85, BEC67 or BEC10, respectively.

The RNA-guided DNA endonuclease comprises as first segment being an RNA-binding portion that interacts with the DNA-targeting RNA and as a second segment being an activity portion that exhibits site-directed enzymatic activity. The first segment interacts with the DNA-targeting RNA and is responsible for the formation of the discussed complex. The second segment harbours the endonuclease domain, which preferably comprises a RuvC domain as described herein above (in particular a RuvC domain of SEQ ID NO: 5 to 7).

As also discussed herein above, the DNA-targeting RNA is the guide RNA. The guide RNA may either be directly introduced into the cells or as a DNA polynucleotide encoding the DNA-targeting RNA. In the latter case, the DNA encoding the guide RNA is generally operably linked to one or more promoter sequences for expression of the guide RNA. For example, the RNA coding sequence can be operably linked to a promoter sequence that is recognized by RNA polymerase III (Pol III) or RNA polymerase II (Pol II). The DNA polynucleotide encoding the DNA-targeting RNA is preferably a vector. Many single gRNA empty vectors (with and without the CRISPR endonuclease) are available in the art. Also, several empty multiplex gRNA vectors are available that can be used to express multiple gRNAs from a single plasmid (with or without the expression of the CRISPR endonuclease). The DNA polynucleotide encodes the DNA-targeting RNA in expressible form.

Likewise, the RNA-guided DNA endonuclease may either be directly introduced into the cells or as a nucleic acid molecule encoding the RNA-guided DNA endonuclease, the latter being preferably a vector of the second aspect. The DNA polynucleotide encodes the RNA-guided DNA endonuclease in expressible form.

As is discussed in greater detail herein above, the RNA-guided DNA endonuclease and the DNA-targeting RNA may also be encoded by the same DNA polynucleotide, such as an all-in one CRISPR-cas vector.

The term "in expressible form" means that the one or more DNA polynucleotides encoding the RNA-guided DNA endonuclease and the DNA-targeting RNA are in a form that ensures that the DNA-targeting RNA is transcribed and that the RNA-guided DNA endonuclease is transcribed and translated into the active enzyme in the cells.

In accordance with a preferred embodiment of the ninth aspect of the invention in case the RNA-guided DNA endonuclease and the DNA-targeting RNA are directly introduced into the cell they are introduced in the form of a ribonucleoprotein complex (RNP).

RNPs are assembled *in vitro* and can be delivered to the cell by methods known in the art, for example, electroporation or lipofection. RNPs are capable to cleave the target site with comparable efficacy as nucleic acid-based (e.g. vector-based) RNA-guided DNA endonucleases (Kim et al. (2014), Genome Research 24(6):1012-1019).

Means for introducing proteins (or peptides) or RNPs into living cells are known in the art and comprise but are not limited to microinjection, electroporation, lipofection (using liposomes), nanoparticle-based delivery, and protein transduction. Any one of these methods may be used.

A liposome used for lipofection is a small vesicle, composed of the same material as a cell membrane (i.e., normally a lipid bilayer e.g. made of phospholipids), which can be filled with one or more protein(s) (e.g. Torchilin VP. (2006), Adv Drug Deliv Rev., 58(14):1532-55). To deliver a protein or RNP into a cell, the lipid bilayer of the liposome can fuse with the lipid bilayer of the cell membrane, thereby delivering the contained protein into the cell. It is preferred that the liposomes used in accordance with invention are composed of cationic lipids. The cationic liposome strategy has been applied successfully to protein delivery (Zelphati et al. (2001). J. Biol. Chem. 276, 35103-35110). As known in the art, the exact composition and/or mixture of cationic lipids used can be altered, depending upon the protein(s) of interest and the cell type used (Felgner et al. (1994). J. Biol. Chem. 269, 2550-2561). Nanoparticle-based delivery of Cas9 ribonucleoprotein and donor DNA for the induction of homology-directed DNA repair is, for example, described in Lee et al. (2017), Nature Biomedical Engineering, 1:889-90.

Protein transduction specifies the internalisation of proteins into the cell from the external environment (Ford et al (2001), Gene Therapy, 8:1-4). This method relies on the inherent property of a small number of proteins and peptides (preferably 10 to 16 amino acids long) to penetrate the cell membrane. The transducing property of these molecules can be conferred upon proteins which are expressed as fusions with them and thus offer, for example, an alternative to gene therapy for the delivery of therapeutic proteins into target cells. Commonly used proteins or peptides being able to penetrate the cell membrane are, for example; the antennapedia peptide, the herpes simplex virus VP22 protein, HIV TAT protein transduction domain, peptides derived from neurotransmitters or hormones, or a 9xArg-tag.

Microinjection and electroporation are well known in the art and the skilled person knows how to perform these methods. Microinjection refers to the process of using a glass micropipette to introduce substances at a microscopic or borderline macroscopic level into a single living cell. Electroporation is a significant increase in the electrical conductivity and permeability of the cell plasma membrane caused by an externally applied electrical field. By increasing permeability, protein (or peptides or nucleic acid sequences) can be introduced into the living cell.

The RNA-guided DNA endonuclease may be introduced into the cells as an active enzyme or as a proenzyme. In the latter case the RNA-guided DNA endonuclease is biochemically changed within the cells (for example by a hydrolysis reaction revealing the active site or changing the configuration to reveal the active site), so that the proenzyme becomes an active enzyme.

Means and methods for the introduction of nucleic acid molecule(s) and DNA-targeting RNA into cells are likewise known in the art and these methods encompass transducing or transfecting cells.

Transduction is the process by which foreign DNA is introduced into a cell by a virus or viral vector. Transduction is a common tool used by molecular biologists to stably introduce a foreign gene into a host cell's genome. Generally, a plasmid is constructed in which the genes to be transferred are flanked by viral sequences that are used by viral proteins to recognize and package the viral genome into viral particles. This plasmid is inserted (usually by transfection) into a producer cell together with other plasmids (DNA constructs) that carry the viral genes required for formation of infectious virions. In these producer cells, the viral proteins expressed by these packaging constructs bind the sequences on the DNA/RNA (depending on the type of viral vector) to be transferred and insert it into viral particles. For safety, none of the plasmids used contains all the sequences required for virus formation, so that simultaneous transfection of multiple plasmids is required to get infectious virions. Moreover, only the plasmid carrying the sequences to be transferred contains signals that allow the genetic materials to be packaged in virions, so that none of the genes encoding viral proteins are packaged. Viruses collected from these cells are then applied to the cells to be altered. The initial stages of these infections mimic an infection with natural viruses and lead to expression of the genes transferred and (in the case of lentivirus/retrovirus vectors) insertion of the DNA to be transferred into the cellular genome. However, since the transferred genetic material does not encode any of the viral genes, these infections do not generate new viruses (the viruses are "replication-deficient"). In the present case transduction may be used to generate cells that comprise the RNA-guided DNA endonuclease in their genome in expressible form.

Transfection is the process of deliberately introducing naked or purified nucleic acids or purified proteins or assembled ribonucleoprotein complexes into cells. Transfection is generally a non-viral based method.

Transfection may be a chemical-based transfection. Chemical-based transfection can be divided into several kinds: transfection using cyclodextrin, polymers, liposomes, or nanoparticles. One of the cheapest methods uses calcium phosphate. HEPES-buffered saline solution (HeBS) containing phosphate ions are combined with a calcium chloride solution containing the DNA to be transfected. When the two are combined, a fine precipitate of the positively charged calcium and the negatively charged phosphate will form, binding the DNA to be transfected on its surface. The suspension of the precipitate is then added to the cells to be transfected (usually a cell culture grown in a monolayer). By a process not entirely understood, the cells take up some of the precipitate, and with it, the DNA. This process has been a preferred method of identifying many oncogenes. Other methods use highly branched organic compounds, so-called dendrimers, to bind the DNA and transfer it into the cell. Another method is the use of cationic polymers such as DEAE-dextran or polyethylenimine (PEl). The negatively charged DNA binds to the polycation and the complex is taken up by the cell via endocytosis. Lipofection (or liposome transfection) is a technique used to inject genetic material into a cell by means of liposomes, which are vesicles that can easily merge with the cell membrane since they are both made of a phospholipid bilayer, as mentioned above. Lipofection generally uses a positively charged (cationic) lipid (cationic liposomes or mixtures) to form an aggregate with the negatively charged (anionic) genetic material. This transfection technology performs the same tasks in terms of transfer into cells as other biochemical procedures utilizing polymers, DEAE-dextran, calcium phosphate, and electroporation. The efficiency of lipofection can be improved by treating transfected cells with a mild heat shock. Fugene is a series of widely used proprietary non-liposomal transfection reagents capable of directly transfecting a wide variety of cells with high efficiency and low toxicity.

Transfection may also be a non-chemical method. Electroporation (gene electrotransfer) is a popular method, where transient increase in the permeability of cell membrane is achieved when the cells are exposed to short pulses of an intense electric field. Cell squeezing enables delivery of molecules into cells via cell membrane deformation. Sonoporation uses high-intensity ultrasound to induce pore formation in cell membranes. This pore formation is attributed mainly to the cavitation of gas bubbles interacting with nearby cell membranes since it is enhanced by the addition of ultrasound contrast agent, a source of cavitation nuclei. Optical transfection is a method where a tiny (~1 µm diameter) hole is transiently generated in the plasma membrane of a cell using a highly focused laser. Protoplast fusion is a technique in which transformed bacterial cells are treated with lysozyme in order to remove the cell wall. Following this, fusogenic agents (e.g., Sendai virus, PEG, electroporation) are used in order to fuse the protoplast carrying the gene of interest with the recipient target cell.

Finally, transfection may be a particle-based method. A direct approach to transfection is the gene gun, where the DNA is coupled to a nanoparticle of an inert solid (commonly gold), which is then "shot" (or particle bombardment) directly into the target cell's nucleus. Hence, the nucleic acid is delivered through membrane penetration at a high velocity, usually connected to microprojectiles. Magnetofection, or magnet-assisted transfection, is a transfection method that uses magnetic force to deliver DNA into target cells. Impalefection is carried out by impaling cells by elongated nanostructures and arrays of such nanostructures such as carbon nanofibers or silicon nanowires which have been functionalized with plasmid DNA.

The method of the ninth aspect of the invention relates to a method for editing (i.e. "mutating") with the RNA-guided DNA endonuclease of the invention a nucleotide sequence at a target site in the genome of a cell. This requires essentially three sequential preconditions: (1) Efficient delivery of the RNA-guided DNA endonuclease-encoding genes or the RNA-guided DNA endonuclease itself into the target cell; (2) efficient expression or presence of the CRISPR-components in the target cell (DNA-targeting RNA and the RNA-guided DNA endonuclease of the sixth aspect); and (3) targeting of the genomic site of interest by CRISPR ribonucleoprotein complexes and repair of the DNA by cell's own repair pathways. Step (3) is automatically carried out in the cell upon the expression of the CRISPR-components in the cell the genome of which is to be edited.

By genome editing a target site may be inserted, deleted, modified (including singe nucleotide polymorphisms (SNPs)) or replaced in the genome of the cell. The target site can be in the coding region of a gene, in an intron of a gene, in a control region of a gene, in a non-coding region between genes, etc. The gene can be a protein coding gene or an RNA coding gene. The gene can be any gene of interest.

In this connection genome editing uses the cell's own repair pathways, including the non-homologous end-joining (NHEJ) or homology directed recombination (HDR) pathway. Once the DNA is cut by the RNA-guided DNA endonuclease, the cell's own DNA repair machinery (NHEJ or HDR) adds or deletes pieces of genetic material or makes changes to the DNA by replacing an existing segment with a customized DNA sequence. Hence, in the CRISPR-Cas system, the CRISPR nuclease makes a double-stranded break in DNA at a site determined by the short (∼20 nucleotide) gRNA which break is then repaired within the cell by NHEJ or HDR. It is preferred that genome editing uses NHEJ. In a different embodiment, it is preferred that genome editing uses HDR.

NHEJ uses a variety of enzymes to directly join the DNA ends in a double-strand break. In contrast, in HDR, a homologous sequence is utilized as a template for the regeneration of missing DNA sequence at the break point. NHEJ is the canonical homology-independent pathway as it involves the alignment of only one to a few complementary bases at most for the re-ligation of two ends, whereas HDR uses longer stretches of sequence homology to repair DNA lesions.

The natural properties of these pathways form the very basis of RNA-guided DNA endonuclease-based genome editing. NHEJ is error-prone and has been shown to cause mutations at the repair site. Thus, if one is able to create a double strand break (DSB) at a desired gene in multiple samples, it is very likely that mutations will be generated at that site in some of the treatments because of errors created by the NHEJ infidelity. On the other hand, the dependency of HDR on a homologous sequence to repair DSBs can be exploited by inserting a desired sequence within a sequence that is homologous to the flanking sequences of a DSB which, when used as a template by the HDR system, would lead to the creation of the desired change within the genomic region of interest. Despite the distinct mechanisms, the concept of the HDR based gene editing is in a way similar to that of homologous recombination-based gene targeting. So, based on these principles if one is able to create a DSB at a specific location within the genome, then the cell's own repair systems will help in creating the desired mutations.

The homologous sequence template for HDR is also referred to herein as "repair template".

Hence, by modifying a nucleotide sequence at a target site in the genome of a cell according to the ninth aspect of the invention a gene may be knocked-out (by introducing per-mature stop codon) or knocked-in (via the repair substrate). It is likewise possible to alter the expression of a gene by the method of the ninth aspect of the invention. For instance, the target site in the genome may be a promoter region changing the promoter region may increase or decrease the expression of the gene being controlled via the target promoter region.

Hence, in accordance with a preferred embodiment of the ninth aspect the method further comprises the introduction of a repair substrate into said cell.

The designs and structures of repair templates being suitable for HDR are known in the art. HDR is error-free if the repair template is identical to the original DNA sequence at the double-strand break (DSB), or it can introduce very specific mutations into DNA. The three central steps of the HDR pathways are: (1) The 5'-ended DNA strand is resected at the break to create a 3' overhang. This will serve as both a substrate for proteins required for strand invasion and a primer for DNA repair synthesis. (2) The invasive strand can then displace one strand of the homologous DNA duplex and pair with the other. This results in the formation of the hybrid DNA, referred to as the displacement loop (D loop). (3) The recombination intermediates can then be resolved to complete the DNA repair process.

HDR templates used, for example, to introduce mutations or insert new nucleotides or nucleotide sequences into a gene require a certain amount of homology surrounding the target sequence that will be modified. Homology arms can be used that start at the CR!SPR-induced DSB. In general, the insertion sites of the modification should be very close to the DSB, ideally less than 10 bp away, if possible. One important point to note is that the CRISPR enzymes may continue to cleave DNA once a DSB is introduced and repaired. As long as the gRNA target site/PAM site remain intact, the CRISPR nuclease will keep cutting and repairing the DNA. This repeated editing may be problematic if a very specific mutation or sequence is to be introduced into a gene of interest. To get around this, the repair template can be designed in such a way that it will ultimately block further CRISPR nuclease targeting after the initial DSB is repaired. Two common ways to block further editing are mutating the PAM sequence or the gRNA seed sequence. When designing a repair template, the size of the intended edit is to be taken into consideration. ssDNA templates (also referred to as ssODNs) are commonly used for smaller modifications. Small insertions/edits may require as little as 30-50 bases for each homology arm, and the best exact number may vary based on the gene of interest. 50-80 base homology arms are commonly used. For example, Richardson et al. (2016). Nat Biotechnol. 34(3):339-44) found that asymmetric homology arms (36 bases distal to the PAM and 91 bases proximal to the PAM) supported HDR efficiencies up to 60 %. Due to difficulties that might be associated with creating ssODNs longer than 200 bases, it is preferred to use dsDNA plasmid repair templates for larger insertions such as fluorescent proteins or selection cassettes into a gene of interest. These templates can have homology arms of at least 800 bp. To increase the frequency of HDR edits based on plasmid repair templates, self-cleaving plasmids can be used that contain gRNA target sites flanking the template. When the CRISPR nuclease and the appropriate gRNA(s) are present, the template is liberated from the vector. To avoid plasmid cloning, it is possible to use PCR-generated long dsDNA templates. Moreover, Quadros et al. (2017) Genome Biol.17;18(1):92) developed Easi-CRISPR, a technique that allows making large mutations and to take advantage of the benefits of ssODNs. To create ssODNs longer than 200 bases, RNA encoding the repair template are *in vitro* transcribed and then reverse transcriptase is used to create the complementary ssDNA. Easi-CRISPR works well in mouse knock-in models, increasing editing efficiency from 1-10 % with dsDNA to 25-50 % with ssODNs. Although HDR efficiency varies across loci and experimental systems, ssODN templates generally provide the highest frequency of HDR edits.

In accordance with a preferred embodiment of the ninth aspect the cell is not the natural host of a gene encoding said RNA-guided DNA endonuclease.

As discussed herein above, the RNA-guided DNA endonucleases of SEQ ID NO: 1, 3 and 29 are developed and optimized using various protein engineering strategies meaning that SEQ ID NO: 1, 3 and 29 are non-naturally occurring sequences with no natural host.

Hence, no known cell is the natural host of SEQ ID NO: 1, 3 and 29.

In accordance with another preferred embodiment of the ninth aspect the cell is a eukaryotic cell, preferably a yeast cell, plant cell or animal cell.

Eukaryotic cells, plant cells and animal cells as well as eukaryotes, plants and animals from which cells may be obtained including preferred examples thereof have been described herein above in connection with the third and fourth aspect of the invention.

Further described but not claimed is a method which further comprises culturing the plant cell or animal cell to produce a plant or non-human animal under conditions in which the RNA-guided DNA endonuclease is expressed and cleaves the nucleotide sequence at the target site to produce a modified nucleotide sequence; and selecting a plant or non-human animal comprising said modified nucleotide sequence.

In this connection the cell(s) into which the components of the CRISPR-Cas system are to be introduced has/have to be a non-human totipotent stem cell or a non-human germ line cell (oocyte and/or sperm) or a collection of stem cells being capable of developing into a complete plant or non-human animal. Means and method for culturing such cell(s) in order to produce a plant or non-human animal are known in the art (see for example, https://www.stembook.org/node/720).

Unless otherwise defined, all technical and scientific terms used herein have the same meaning as commonly understood by one of ordinary skill in the art to which this invention belongs. In case of conflict, the patent specification including definitions, will prevail.

Also described herein but not claimed are modified cells that have been produced by the method according to the ninth aspect of the invention for use in treatment of a disease in a subject.

The modified cells are preferably modified T lymphocytes and the disease to be treated is preferably cancer (Stadtmauer et al., Science 28 Feb 2020:Vol. 367, Issue 6481, eaba7365).

The cells to be modified by the method of the ninth aspect of the invention are preferably obtained from the subject to be treatment and then the modified cells are used in accordance with the tenth aspect of the invention.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show:
**Figure 1****:** Schematic figure visualizing the Ade2 knockout strategy in *S.cerevisiae* S288c for BEC85, BEC67 and BEC10 in comparison to SpCas9
**Figure 2****:** Exemplary culture plates showing *S.cerevisiae* S228c colonies 48h after transformation to visualize the different molecular mechanism of BEC85, BEC67 and BEC10 in comparison to SpCas9
**Figure 3****:** Exemplary culture plates showing *S.cerevisiae* S228c colonies 48h after transformation (incubated at 30°C) to visualize the colony reduction and genome editing efficiency of BEC family nucleases in comparison to the next neighbor sequences SuCms1 and SeqID63. Orange colonies, a mixture of edited and unedited cells, are marked with an arrow.
**Figure 4****:** Exemplary culture plates showing *S.cerevisiae* S228c colonies 48h after transformation (incubated at 21°C) to visualize the colony reduction and genome editing efficiency of BEC family nucleases at lower temperatures (21°C) in comparison to the next neighbor sequences SuCms1 and SeqID63
**Figure 5****:** Exemplary culture plates showing *E. coli BW25113* colonies 48h after transformation (incubated at 37°C) to visualize the colony depletion efficiency of BEC family nucleases at higher temperatures (37°C) in comparison to the next neighbor sequences SuCms1 and SeqID63

The examples illustrate the invention.

### EXAMPLES

### Example 1: Identification and engineering of the BEC family nucleases

Metagenomic sequences with the potential to work as novel genome editing nucleases were in silico identified in various habitats sequenced in house (Burstein et al., Nature (2017) 542, 237-241. As none of these sequences showed intrinsic DNA targeting efficiencies sufficient for genome editing, random shuffling of related sequences was performed (Coco et al., Nat Biotechnol (2001) 19, 354-359) and the randomly created chimeric sequences were additionally optimized using random mutagenesis (McCullum et al., Methods Mol Biol. (2010) 634, 103-9). In the final step, numerous mutagenized chimeric sequences were screened to evaluate their DNA targeting activity.

Using this random and non-rational approach, three sequences (BEC85, BEC67 and BEC10) were successfully identified showing a strong DNA targeting activity potentially sufficient for genome editing approaches. Despite using a random approach, surprisingly all three identified and engineered amino acid sequences share a sequence identity of ≈ 95 % to each other. Based on this sequence identity and their unique DNA targeting mechanism (see Example 3) they are classified herein as a new family of CRISPR nucleases (BEC family: BRAIN Engineered Cas proteins).

### Example 2: Construction of a functional genome editing system comprising nucleases of the BEC-family, Cms1 family and spCas9

### 2.1 CRISPR/BEC and Cms1 vector systems for genome editing in S. cerevisiae S288c

The necessary genetic elements for constitutive expression of the novel CRISPR nucleases of the invention BEC85, BEC67 and, BEC10 as well as two known Cms1 family CRISPR nucleases, SuCms1 (Begemann et al. (2017), bioRxiv) and SeqID63 (WO2019/030695), and for the guide RNA (gRNA) transcription were provided in an all-in-one CRISPR/BEC85, CRISPR/BEC67, CRISPR/BEC10, CRISPR/SuCms1 or CRISPR/SeqID63 vector system.

In the following, the construction of the CRISPR/BEC10 vector system is described. The CRISPR/BEC85 and CRISPR/BEC67, CRISPR/SuCms1 and CRISPR/SeqID63 vector systems were constructed in an analogous approach.

### Design of the BEC10 protein expression cassette

The synthetic 3696 bps BEC10 nucleotide sequence was codon optimized for expression in S. *cerevisiae* S288c, using a bioinformatics application provided by the gene synthesis provider GeneArt (Thermo Fisher Scientific, Regensburg, Germany), SEQ ID NO: 30. Additionally, the DNA nuclease coding sequence was 5' extended by a sequence encoding a SV40 nuclear localization signal (NLS) SEQ ID NO: 11 (Kalderon et al., Cell 39 (1984), 499-509). For protein expression, the resulting synthetic 3723 bps gene was fused to the constitutive S. *cerevisiae* S288c Tpi1 promotor (SEQ ID NO: 12) and the S. *cerevisiae* S288c Cps1 terminator (SEQ ID NO: 13). The final BEC10 protein expression cassette was inserted by Gibson Assembly Cloning (NEB, Frankurt, Germany) into an *E. coli*/*S. cerevisiae* shuttle vector, containing all necessary genetic elements for episomal propagation and selection of recombinant *E. coli* and S. *cerevisiae* cells:
For vector propagation and selection of recombinant *E. coli* cells, the plasmid contained the pUC derived high-copy ColE1 origin of replication and the kanMX marker gene under the control of the synthetic Em7 promotor (SEQ ID NO: 14) conferring kanamycin resistance. The CEN6 centromere (SEQ ID NO: 15) from *S*. *cerevisiae* S288c allowed episomal replication of the shuttle plasmid in *S*. *cerevisiae* cells. For selection of transformed *S*. *cerevisiae* cells the bifunctional bacterial/yeast promotor structure upstream to the kanMX marker gene (SEQ ID NO: 16) contained the *S*. *cerevisiae* S288c Tef1 promotor sequence (SEQ ID NO: 17).

### Design of the guide RNA (gRNA) expression cassette

The expression of the chimeric gRNA for specific Ade2 gene targeting by BEC10 DNA nuclease was driven by the SNR52 RNA polymerase III promotor (SEQ ID NO: 18) with a SUP4 terminator sequence (SEQ ID NO: 19), (DiCarlo et al., NAR (2013), 41, 4336 - 4343). The chimeric gRNA was composed of a constant 19 bps BEC family Stem-Loop Sequence (SEQ ID NO: 9 or SEQ ID NO: 10; both stem loop sequences are interchangeable between all three BEC family nucleases leading to comparable results) fused to the Ade2 target-specific 24 bps spacer sequence (SEQ ID NO: 20). The target spacer sequence was identified in the *S*. *cerevisiae* S288c Ade2 gene downstream to the nuclease BEC10 specific PAM motive 5'-TTTA-3'.

The complete RNA expression cassette composed of the SNR52 RNA polymerase III promotor, the designed chimeric gRNA and the SUP4 terminator sequence was provided as a synthetic gene fragment by GeneArt (Thermo Fisher Scientific, Regensburg, Germany).

The construction of the all-in-one CRISPR/BEC10 vector system was completed by cloning the synthetic RNA expression cassette in the prepared *E. coli*/*S. cerevisiae* shuttle vector, containing the BEC10 DNA nuclease expression cassette. The construction of the final CRISPR/BEC10 vector system was mediated by Gibson Assembly Cloning (NEB, Frankfurt, Germany).

The identity of all cloned DNA elements was confirmed by Sanger-Sequencing at LGC Genomics (Berlin, Germany).

### CRISPR/BEC10 all-in-one-vector system

The complete nucleotide sequence of the constructed CRISPR/BEC10 vector system is provided as SEQ ID NO: 31.

### CRISPR/BEC85 all-in-one-vector system

The complete nucleotide sequence of the constructed CR!SPR/BEC85 vector system is provided as SEQ ID NO: 21.

### CRISPR/BEC67 all-in-one-vector system

The complete nucleotide sequence of the constructed CRISPR/BEC67 vector system is provided as SEQ ID NO: 22.

### CRISPR/SuCms1 all-in-one-vector system

The complete nucleotide sequence of the constructed CRISPR/SuCms1 vector system is provided as SEQ ID NO: 32.

### CRISPR/SeqlD63 all-in-one-vector system

The complete nucleotide sequence of the constructed CRISPR/SeqID63 vector system is provided as SEQ ID NO: 33.

### 2.2 Design of a homology directed repair template (HDR-template)

The 838 bps Ade2 BEC85, BEC67, BEC10, SuCms1 and SeqlD63 HDR-template was designed to generate a site-specific deletion of 29 bps in the chromosomal S. cerevisiae S288c Ade2 gene by homologous recombination. Within the HDR-template, the introduced Ade2 gene deletion was flanked by 407 bps and 429 bps sequences homologous to the chromosomal target region. Additionally, the HDR-fragment created a new recognition sequence for the restriction endonuclease EcoRI at the deleted Ade2 genome site. The successful recombination event mediated by the designed HDR-template abolished the already described PAM and protospacer region (SEQ ID NO: 20) in the chromosomal Ade2 gene to prevent the programmed gRNA/ BEC85, BEC67 or BEC10 DNA nuclease complex to target the *S*. *cerevisiae* S288c genome again. Furthermore, the introduced gene deletion resulted in Ade2 mutant clones, which were easily recognized by the red color of the colonies, since the mutant cells, deprived of adenine, accumulate red purine precursors in their vacuoles (Ugolini et al., Curr Genet (2006), 485-92).

The complete sequence of the Ade2 HDR-template for BEC85, BEC67, BEC10, SuCms1 and SeqID63 is provided as SEQ ID NO: 23.

### 2.3 CRISPR/SpCas9 vector system for genome editing in S. cerevisiae S288c

The necessary genetic elements for constitutive expression of SpCas9 (*S. pyogenes* Cas9) DNA nuclease and for single guide RNA transcription were provided in an all-in-one CRISPR/SpCas9 vector system.

### Design of the SpCas9 protein expression cassette

Based on the published SpCas9 nucleotide sequence from *Streptococcus pyogenes,* (Deltcheva et al., Nature 471 (2011), 602-607) DNA synthesis of the codon optimized SpCas9 coding sequence was ordered at GeneArt (Thermo Fisher Scientific, Regensburg, Germany), (SEQ ID NO: 24) for expression in *S*. *cerevisiae* S288c. For nuclear translocation, the SpCas9 DNA nuclease coding sequence was 5' extended by a sequence encoding a SV40 nuclear localization signal (NLS) (SEQ ID NO: 11). Following the described protein expression strategy for BEC10 DNA nuclease, the resulting synthetic 4134 bps SpCac9 gene was fused to the constitutive *S*. *cerevisiae* S288c Tpi1 promotor (SEQ ID NO: 12) and the *S. cerevisiae* S288c Cps1 terminator (SEQ ID NO: 13). The final SpCas9 protein expression cassette was inserted by Gibson Assembly Cloning (NEB, Frankurt, Germany) into an *E. coli*/*S. cerevisiae* shuttle vector, harbouring the identical genetic elements for propagation and selection as already described for the CRISPR/BEC10 vector system.

### Design of the guide RNA expression (gRNA) cassette

The expression of the chimeric gRNA for specific Ade2 gene targeting by SpCas9 DNA nuclease was driven by the SNR52 RNA polymerase III promotor (SEQ ID NO: 18) with a SUP4 terminator sequence (SEQ ID NO: 19). The chimeric guide RNA was composed of the Ade2 target-specific 20 bps spacer sequence (SEQ ID NO: 25) fused to the 76 bps SpCas9 specific sgRNA sequence (SEQ ID NO: 26). The target spacer sequence was identified in the *S*. *cerevisiae* S288c Ade2 gene upstream to the nuclease SpCas9 specific PAM motive 5'-AGG-3'

The complete RNA expression cassette composed of the SNR52 RNA polymerase III promotor, the designed chimeric guide RNA and the SUP4 terminator sequence was provided as a synthetic DNA fragment by GeneArt (Thermo Fisher Scientific, Regensburg, Germany).

To generate the final CRISPR/SpCas9 vector system, the synthetic RNA transcription cassette was cloned by Gibson Assembly Cloning (NEB, Frankfurt, Germany) into the prepared *E. coli*/*S. cerevisiae* shuttle vector, containing the SpCas9 DNA nuclease expression cassette. The identity of all cloned DNA elements was confirmed by Sanger-Sequencing at LGC Genomics (Berlin, Germany).

### CRISPR/SpCas9 all-in-one-vector system

The complete nucleotide sequence of the constructed CRISPR/SpCas9 vector system is provided as SEQ ID NO: 27.

### 2.4 Design of a homology directed repair template

The synthetic 832 bps Ade2 SpCas9 HDR-template was designed to generate a site-specific deletion of 26 bps in the chromosomal *S*. *cerevisiae* S288c Ade2 gene by homologous recombination. Within the HDR-template, the introduced Ade2 gene deletion was flanked by 402 bps and 428 bps sequences homologous to the chromosomal target region. The successful recombination event mediated by the designed HDR-template abolished the already described PAM and protospacer region (SEQ ID NO: 25) in the chromosomal Ade2 gene to prevent the programmed gRNA / SpCas9 DNA nuclease complex to target the *S*. *cerevisiae* S288c genome again. Furthermore, the introduced gene deletion resulted in Ade2 mutant clones, that could be easily recognized by red color of the colonies, since the mutant cells, deprived of adenine, accumulate red purine precursors in their vacuoles (Ugolini et al., Curr Genet (2006), 485-92).

The nucleotide sequence of the 832 bps Ade2 HDR-template is provided as SEQ ID NO: 28.

### 2.5 Saccharomyces cerevisiae cultivation and transformation

### Transformation of competent S.cerevisiae S288c cells

Preparation and transformation of competent *S*. *cerevisiae S288c* cells were performed as described by Gietz & Schiestl, Nature Protocols (2007), 2, 31 - 34. In brief, a single colony of *S*. *cerevisiae* S288c was inoculated in 25 ml 2x YPD medium and incubated for 14 to 16 h at 30°C on a horizontal shaker at 200rpm. Overnight grown pre-cultures were diluted into fresh 250 ml 2x YPD medium to an optical density at 600 nm (OD600) of 0.5. The inoculated medium was incubated at 30°C on a horizontal shaker at 200 rpm until the culture reached an optical density at OD600 of 2.0 to 8.0. Cells were transferred into 5 x 50 ml conical tubes and harvested by centrifugation for 5 min and 3000 x g. Pelleted cells from 250 ml culture were resuspended in 125 ml water and centrifuged for 5 min and 3000 x g. Pelleted cells were resuspended in 2.5 ml water. After a further centrifugation step for 5 min and 3000 x g, the pelleted cells were finally resuspended in 2.5 ml "frozen competent cell solution" (5 % v/v glycerol and 10 % v/v DMSO). Aliquots of 50 µ! competent cells were stored at - 80°C until use. For transformation procedure, aliquots of competent cells were thawed for 30 sec at 37°C following centrifugation for 2 min at 11.600 x g. The supernatant was removed and the cell pellet was resuspended in 360 µl transformation-mix composed of 1 µg pScCEN plasmid derivatives and 500 ng HDR-template provided in 14 µl water, 260 µl 50 % w/v PEG 3350, 36 µl 1M Li-Acetat and 50 µl single-stranded carrier DNA. Prepared cells were heat-shocked at 42°C for 45 min with mixing every 15 min. Following the heat shock step, transformed cells were pelleted for 30 sec at 13.000 x g by centrifugation, the supernatant was removed. For recovery, the cell pellet was resuspended in 1 ml YPD. The cell suspension was transferred into a 5 ml tube and incubated for 3 h at 30°C on a horizontal shaker at 200 rpm. Finally, the transformed cells were plated on selective agar plates containing 50 µg/ml geneticin (G418) and incubated at least for 2 days at 30°C.

### 2.6 CRISPR/BEC E. coli and Cms1 E. coli vector systems for genome editing in E. coli BW25113

The necessary genetic elements for constitutive expression of the BEC10, SuCms1 or SeqID63 CRISPR nucleases and for the guide RNA (gRNA) transcription were provided in an all-in-one CRISPR/BEC10_E. coli (SEQ ID NO: 34), CRISPR/SuCms1_E. coli (SEQ ID NO: 35) or CRISPR/SeqID63_E. coli (SEQ ID NO: 36) vector system.

In the following, the construction of the CRISPR/BEC10_E. coli vector system is described. The CRISPR/SuCms1_Coli and CRISPR/SeqID63_Coli vector systems were constructed in an analogous approach.

### Design of the BEC10 Coli protein expression cassette

The synthetic 3696 bps BEC10 nucleotide sequence was codon optimized for expression in *E. coli BW25113,* using a bioinformatics application provided by the gene synthesis provider GeneArt (Thermo Fisher Scientific, Regensburg, Germany), SEQ ID NO: 37. For protein expression, the resulting synthetic gene was fused to the inducible araBAD promotor (SEQ ID NO: 38) and the fdt terminator (SEQ ID NO: 39). The final BEC10_*E*. *coli* protein expression cassette was inserted by Gibson Assembly Cloning (NEB, Frankurt, Germany) into an *E. coli* shuttle vector, containing all necessary genetic elements for episomal propagation and selection of recombinant *E. coli* cells.

### Design of the guide RNA (gRNA) expression cassette

The expression of the chimeric gRNA for specific rpoB gene targeting by BEC10 DNA nuclease was driven by the SacB RNA polymerase III promotor (SEQ ID NO: 40) and terminated using a rrnB terminator sequence (SEQ ID NO: 41). The chimeric gRNA was composed of a constant 19 bps BEC family Stem-Loop Sequence (SEQ ID NO: 9 or SEQ ID NO: 10; both stem loop sequences are interchangeable between all three BEC family nucleases leading to comparable results) fused to the rpoB target-specific 24 bps spacer sequence (SEQ ID NO: 42).The target spacer sequence was identified in the *E. Coli BW25113* rpoB gene downstream to the nuclease BEC10 specific PAM motif 5'-TTTA-3'.

The complete RNA expression cassette composed of the SacB RNA polymerase III promotor, the designed chimeric gRNA and the rrnB terminator sequence was provided as a synthetic gene fragment by GeneArt (Thermo Fisher Scientific, Regensburg, Germany).

The construction of the all-in-one CRISPR/BEC10_E. coli vector system was completed by cloning the synthetic RNA expression cassette in the prepared *E. coli* shuttle vector, containing the BEC10_Coli DNA nuclease expression cassette. The construction of the final CRISPR/BEC10_E. coli vector system was mediated by Gibson Assembly Cloning (NEB, Frankfurt, Germany).

The identity of all cloned DNA elements was confirmed by Sanger-Sequencing at LGC Genomics (Berlin, Germany).

### CRISPR/BEC10 E. coli all-in-one-vector system

The complete nucleotide sequence of the constructed CRISPR/BEC10_Coli vector system is provided as SEQ ID NO: 34.

### CRISPR/SuCms1 E. coli all-in-one-vector system

The complete nucleotide sequence of the constructed CRISPR/SuCms1_Coli vector system is provided as SEQ ID NO: 35.

### CRISPR/SeqID63 E. coli all-in-one-vector system

The complete nucleotide sequence of the constructed CRISPR/SuCms1_Coli vector system is provided as SEQ ID NO: 36.

### 2.7 E. coli cultivation and transformation

### Transformation of competent E. coli BW25113 cells

In brief, a single colony of *E. coli BW25113* was inoculated in 5 ml LB-Kan medium and incubated for 12 to 14 h at 37°C on a horizontal shaker at 200rpm. Overnight grown pre-cultures were diluted into fresh 60 ml LB medium to an optical density at 600 nm (OD600) of 0.06. The inoculated medium was incubated at 30°C on a horizontal shaker at 200 rpm until the culture reached an optical density at OD600 of 0.2. 600 µl. 20 % arabinose was added and the cells were incubated at 30°C at 200rpm until the culture reached an optical density at OD600 of 0.5. Cells were transferred into a 50 ml conical tube and harvested by centrifugation at 4°C for 5 min and 4000 x g. Pelleted cells from 50 ml culture were resuspended in 60 ml water and centrifuged at 4°C for 5 min and 4000 x g.

A washing procedure was performed and the cells were resuspended in 30 ml 10 % glycerin following a centrifugation at 4°C for 5 min and 4000 x g. In a second washing step, the cells were resuspended in 6 ml 10 % glycerin following a centrifugation at 4°C for 5 min and 4000 x g. In the final step, the cells were resuspended in 150 µ! 10 % glycerin. Aliquots of 25 µ! competent cells were stored at - 80°C until use. For transformation procedure, aliquots of competent cells were thawed and 50 ng plasmid DNA was added. Prepared cells were electroporated using 1800 V, 25 µF, 200 Ohm for 5 msec. Subsequently, 975 µL of NEB^{®} 10-beta/Stable Outgrowth Medium was added and 100 µl of the suspension was plated on selective agar plates.

### 2.8 DNA techniques

Plasmid isolation, enzymatic manipulation of DNA and agarose gel electrophoresis were performed according to standard procedures. The Thermo Fisher Scientific Phusion Flash High-Fidelity PCR system (Thermo Fisher Scientific, Darmstadt, Germany) was used for PCR amplifications. All oligonucleotides used in this work were synthesized by biomers.net (Ulm, Germany) or Eurofins Scientific (Ebersberg, Germany). The DNA Clean and Concentrator Kit and the ZymoClean Gel DNA Recovery Kit (Zymo Research, Freiburg, Germany) were used for purifications from agarose and enzymatic reactions. The identity of all cloned DNA-fragments was confirmed by Sanger sequencing technology at LGC Genomics (Berlin, Germany).

Purified genomic DNA from *S*. *cerevisiae* S288c cells was isolated using the Zymo Research's YeaStar Genomic DNA Kit (Zymo Research, Freiburg, Germany) according to the manufacturer's instructions. Zymolyase-digestion of Yeast cell wall was performed for 60 min at 37°C, purified genomic DNA was eluted in 60 µl 5 mM Tris/HCl pH 8.5.

### Example 3: Functional Characterization of BEC85, BEC67 and BEC10 in comparison to spCas9 in Saccharomyces cerevisiae (S. cerevisiae)

### 3.1. Experimental Setup

In this example, the CRISPR/BEC85 (SEQ ID NO: 21), CRISPR/BEC67 (SEQ ID NO: 22) or CRISPR/BEC10 (SEQ ID NO: 31) vector system and the corresponding homology directed repair template (SEQ ID NO: 23) were used to knock out the Ade2 gene in *S*. *cerevisiae* S288C. In comparison to the experiments carried out using BEC85, BEC67 or BEC10, similar experiments were conducted using the CRISPR/SpCas9 construct (SEQ ID NO: 27) and the corresponding homology directed repair template (SEQ ID NO: 28) to demonstrate the functionality of BEC type CRISPR nucleases.

Ade2 is a non-essential gene of *Saccharomyces cerevisiae* but a knockout leads to a red phenotype of the colonies, since the mutant cells accumulate red purine precursors in their vacuoles (Ugolini et al., Curr Genet (2006), 485-92) . Due to this easy readout, the knockout of the Ade2 gene can be utilized as a screening system to monitor the ability of CRISPR Cas proteins to function as a genome-editing tool.

In this approach, a CRISPR Cas directed introduction of a homology directed repair template leading to a site-specific deletion eliminating the PAM and spacer sequence was used. Furthermore, a frame shift mutation was introduced by the homology directed repair template leading to a knockout of the Ade2 gene to visualize the DNA cleavage activity of BEC85, BEC67 and BEC10 in comparison to SpCas9 (the most commonly used Cas protein in science and pharma).

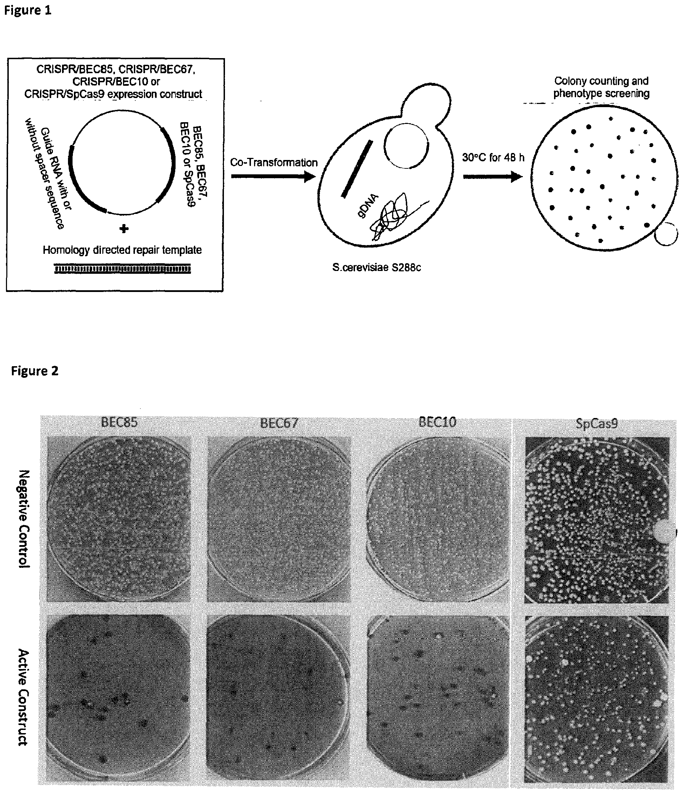

The used Ade2 knockout strategy in *S*. *cerevisiae* S288c for BEC85, BEC67, BEC10 and SpCas9 is shown schematically in Fig. 1.

In summary, the CRISPR/BEC85, CRISPR/BEC67, CRISPR/BEC10 or CRISPR/SpCas9 expression constructs and the corresponding homology directed repair template were transformed into S. *cerevisiae* S288c cells and plated as described in Example 2.5.

In parallel, negative control experiments using the CRISPR/BEC85, CRISPR/BEC67, CRISPR/BEC10 or CRISPR/SpCas9 expression constructs lacking a spacer sequence targeting the Ade2 gene were performed to demonstrate the dependency of the Cas proteins to be guided to the target DNA region by a specific spacer.

After transformation and 48 h incubation at 30°C the culture plates were analyzed by counting the number of grown colonies and by the evaluation of their phenotype (red or white).

### 3.2 Results

The results are summarized in Table 1 and exemplary plates are shown in Figure 2.

All experiments were carried out in 5 biological replicates and the results obtained from these replicates were combined to visualize the genome editing efficacy of BEC type CRISPR nucleases.

**Table 1: Summary of the results of 5 experiments (plates) for each experimental setup using the Ade2 knockout strategy in S. cerevisiae S288c for BEC85, BEC67, BEC10 and SpCas9 (cumulated colony numbers)**

| | **BEC85** | **BEC85 Negative Control** | **BEC67** | **BEC67 Negative Control** | **BEC10** | **BECIO Negative Control** | **SpCas9** | **SpCas9 Negative Control** |
|---|---|---|---|---|---|---|---|---|
| **White Phenotype** | 9 | 6643 | 21 | 9136 | 19 | 8021 | 1182 | 5831 |
| **Red Phenotype** | 82 | 14 | 45 | 16 | 155 | 14 | 2575 | 11 |
| **Total Colonies** | 91 | 6657 | 66 | 9152 | 174 | 8035 | 3757 | 5842 |
| **Editing Efficiency** (%) | 90 | 0.2 | 68 | 0.2 | 89 | 0.2 | 68 | 0.2 |

### CRISPR/SpCas9

Cells transformed with the negative control constructs (CRISPR/SpCas9 (without spacer) + homology directed repair template) showed 5831 white and 11 red colonies demonstrating that the SpCas9 protein did not target the DNA of the Ade2 gene due to the missing spacer sequence. Therefore, 99.8 % of the colonies showed a wild type phenotype (white). Furthermore, 11 colonies showed a knockout phenotype (red) due to natural homological recombination events where the homology directed repair template integrates into the Ade2 gene locus.

In contrast to this, the active construct (CRISPR/SpCas9 (with a spacer targeting the Ade2 gen) + homology directed repair template) led to 1182 white and 2575 red colonies. Thus, demonstrating the molecular mechanism and efficacy of SpCas9 with 68 % of edited colonies in comparison to the negative control where only 0.2 % of the colonies were edited.

### CRISPR/BEC85, CRISPR/BEC67 and CRISPR/BEC10

Surprisingly, the same experimental setup using the BEC85, BEC67 or BEC10 sequence led to completely different results as compared to SpCas9.

Cells transformed with the BEC10 negative control constructs (CRISPR/BEC10 (without spacer) + homology directed repair template) showed 8021 white and 14 red colonies demonstrating that the BEC10 protein did not target the DNA of the Ade2 gene due to the missing spacer sequence. Therefore, 99.8 % of the colonies showed a wild type phenotype (white). Furthermore, 14 colonies showed a knockout phenotype (red) due to natural homological recombination events where the homology directed repair template integrates into the Ade2 gene locus. Similar results were obtained using the BEC85 (6643 wild type (white) and 14 knockout (red) colonies) and BEC67 (9136 wild type (white) and 16 knockout (red) colonies) negative control construct.

In contrast to this, the active BEC10 construct (CRISPR/BEC10 (with a spacer targeting the Ade2 gen) + homology directed repair template) led to a significant overall reduction of visible colonies (174) compared to the negative control (8035) and also compared to the active SpCas9 approach (3757). However, 155 out of these 174 colonies showed an Ade2 knock out phenotype (red) leading to an editing efficiency of 89 %. Using the active BEC85 or BEC67 construct similar results were observed. BEC85: Significant colony reduction down to 91 with 82 red and 9 white colonies leading to an editing efficacy of 90 %

BEC67: Significant colony reduction down to 68 with 45 red and 21 white colonies leading to an editing efficacy of 68 %.

Taken together, the results obtained using the experimental setups with SpCas9, BEC85, BEC67 and BEC10 surprisingly showed a completely different molecular genome editing mechanism of BEC type CRISPR nucleases in comparison to classical CRISPR Cas nucleases. In contrast to SpCas9, which assists homologous recombination by introducing a RNA directed double strand break, BEC85, BEC67 and BEC10 mediated editing leads to a strong overall clone reduction in connection with a significant enrichment of cells that successfully accomplished homologous recombination.

Even though BEC85, BEC67 and BEC10 show a novel molecular mechanism, the results obtained in this example prove the capability of BEC type CRISPR nucleases to function as a novel genome editing tool by site directed, highly efficient homology directed recombination.

### Example 4: Evaluation of the genome editing activity and efficiency of BEC family nucleases in comparison towards their next neighbor sequences SuCms1 and SeqID63.

Example 4 demonstrates that the novel BEC family nucleases of the present invention are superior as compared to their closest known relatives SuCms1 (Begemann et al. (2017), bioRxiv) and SeqID63 (WO2019/030695) based on comparative experiments.

### 4.1. Experimental Setup

In this example, the CRISPR/BEC10 (SEQ ID NO: 31), CRISPR/SuCms1(SEQ ID NO: 32) or CRISPR/SeqID63 (SEQ ID NO: 33) vector system and the corresponding homology directed repair templates (SEQ ID NO: 23) were used to knock out the Ade2 gene in *S*. *cerevisiae* S288C. The example directly compares the genome editing efficiency of the BEC family nucleases with their next neighbor sequences SuCms1 (Begemann et al. (2017), bioRxiv) and SeqID63 (WO2019/030695).

The experiments were carried out as described in the section 3.1, *supra,* of the examples.

### 4.2 Results

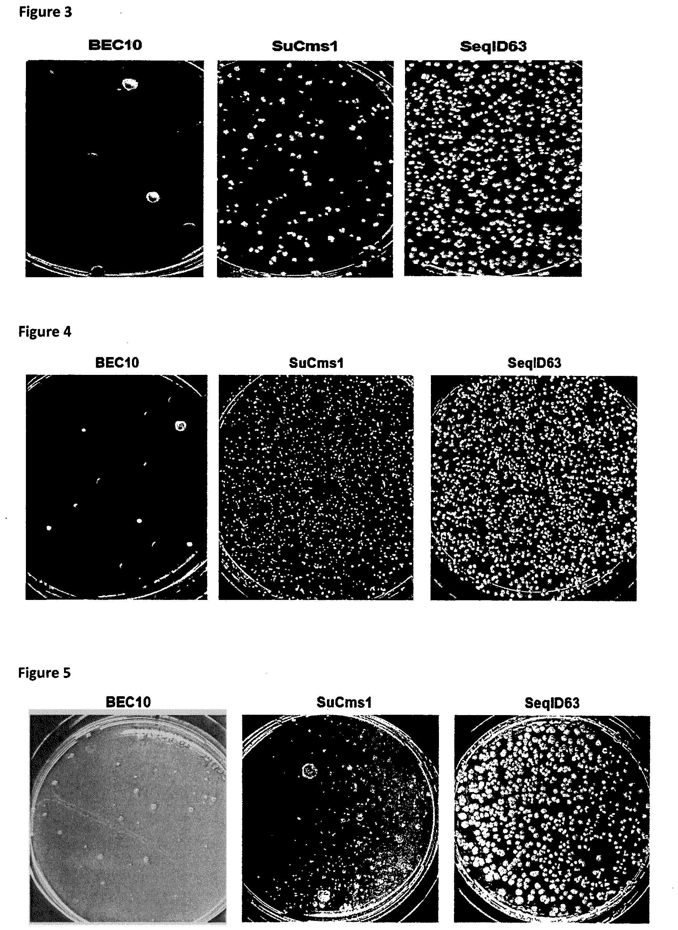

The results are summarized in Table 2 and exemplary plates are shown in Figure 3.

All experiments were carried out in 5 biological replicates and the results obtained from these replicates were combined to visualize the genome editing efficacy of BEC10 in comparison to the prior art nucleases SuCms1 and SeqID63.

**Table 2: Summary of the results of 5 experiments (plates) for each experimental setup using the Ade2 knockout strategy in S. cerevisiae S288c for BEC10, SuCms1 and SeqID63 (cumulated colony numbers) at 30°C**

| | **BEC10** | **SuCms1** | **SeqID63** |
|---|---|---|---|
| **White Phenotype** | 11 | 623 | 5231 |
| **Red Phenotype** | 59 | 19 | 8 |
| **Orange Phenotype** | 0 | 14 | 0 |
| **Total Colonies** | 70 | 656 | 5239 |
| **Editing Efficiency (%)** | 84 | 5 | 0.2 |

### CRISPR/SuCms1

Cells transformed with the active construct (CRISPR/SuCms1 + homology directed repair template) showed 623 white, 19 red and 14 orange colonies (the orange colonies in Figure 3 are marked with an arrow) leading to an editing efficiency of 5 % (if the orange colonies are counted as successfully edited cells). However, further analysis of the orange colonies showed that these clones contained a mixture of successfully edited and not edited (i.e. wild-type) cells leading to an editing efficiency of fully edited colonies of only 3 %.

### CRISPR/SeqID63

Cells transformed with the active construct (CRISPR/SeqID63 + homology directed repair template) showed 5231 white and 8 red colonies leading to an editing efficiency of only 0.2 %. The total colony numbers and the editing efficacy is comparable to the negative control results shown in Example 3 demonstrating that the SeqID63 does not show any nuclease activity.

### CRISPR/BEC10

Cells transformed with the active construct (CRISPR/BEC10 + homology directed repair template) showed 11 white and 59 red colonies leading to a very high editing efficacy of 84 %, which is comparable to the editing efficacy as obtained in Example 3 for BEC85, BEC67 and BEC10.

### Summary

The results obtained in Example 4 show that BEC10 and the other BEC family nucleases as well as BEC85 and BEC67 (in view of the results as described Example 3) have the same DNA targeting mechanism and show that all three have very high and comparable editing efficiencies. Furthermore, BEC family nucleases show a significantly stronger colony reduction and a significantly superior editing efficiency in comparison to their next neighbor sequences SuCms1 and SeqID63. In contrast to the SuCms1 nuclease, which shows an editing efficiency of 5 % (also noting that out of the 33 edited clones 14 where just partly edited), BEC10 shows an editing efficacy of 84 %, BEC85 of 90 % and BEC67 of 68 % (see Example 3). Furthermore, SeqID63 does not show any nuclease activity at all.

### Example 5: Evaluation of the genome editing activity and efficiency of BEC family nucleases in comparison to their next neighbor sequences SuCms1 and SeqID63 at different temperatures (21°C and 37°C)

For many biotechnological and pharmaceutical applications experiments have to be carried out at specific temperatures to meet the requirements for the used organism and to ensure the best performance and reproducible results. The optimal temperature for most organisms used for biotechnological, agricultural and pharmaceutical applications is between 21°C and 37°C. To demonstrate the performance of the BEC nucleases of the invention in this temperature range experiments were carried out using S. cerevisiae (21°C) and *E. coli* (37°C) in comparison to the next neighbor sequences SuCms1 (Begemann et al. (2017), bioRxiv) and SeqID63 (WO2019/030695).

### 5.1. Experimental Setup (S. cerevisiae 21°C)

In this example, the CRISPR/BEC10 (SEQ ID NO: 31), CRISPR/SuCms1(SEQ ID NO: 32) or CRISPR/SeqID63 (SEQ ID NO: 33) vector system and the corresponding homology directed repair template (SEQ ID NO: 23) were used to knock out the Ade2 gene in *S*. *cerevisiae* S288C. Cells were incubated at 21°C to demonstrate the editing efficiency of the BEC family nucleases at low temperatures in direct comparison towards their next neighbor sequences SuCms1 and SeqID63.

*S. cerevisiae* cultivation and transformation was carried out as described in section 2.5 of the examples except for the cultivation temperature that was altered from 30°C to 21°C.

The experiments were carried out like described in the section 3.1 of the examples.

### 5.2 Results

The results are summarized in Table 3 and exemplary plates are shown in Figure 4.

All experiments were carried out in 5 biological replicates and the results obtained from these replicates were combined to visualize the genome editing efficacy of BEC10 in comparison to SuCms1 and SeqID63.

**Table 3: Summary of the results of 5 experiments (plates) for each experimental setup using the Ade2 knockout strategy in S. cerevisiae S288c for BEC10, SuCms1 and SeqID63 (cumulated colony numbers) at 21°C**

| | **BEC10** | **SuCms** | **SeqID63** |
|---|---|---|---|
| **White Phenotype** | 23 | 8740 | 10240 |
| **Red Phenotype** | 42 | 28 | 18 |
| **Total Colonies** | 65 | 8768 | 10258 |
| **Editing Efficiency (%)** | 65 | 0.3 | 0.2 |

### CRISPR/SuCms1

Cells transformed with the active construct (CRISPR/SuCms1 + homology directed repair template) showed 8740 white and 28 red colonies leading to an editing efficiency of 0.3 %, which is just slightly above the editing efficiency of the negative control experiments (0.2 %) and significantly decreased in comparison to the results obtained at 30°C (Example 4).

### CRISPR/SeqlD63

Cells transformed with the active construct (CRISPR/SeqID63 + homology directed repair template) showed 10240 white and 18 red colonies leading to an editing efficiency of 0.2 %. The total colony numbers and the editing efficacy is comparable to the negative control results shown in Example 4 demonstrating that SeqID63 does not show any nuclease activity.

### CRISPR/BEC10

Cells transformed with the active construct (CRISPR/BEC10 + homology directed repair template) showed 23 white and 42 red colonies still leading to a high editing efficacy of 64 %, thereby demonstrating that genome editing using BEC type CRISPR nucleases leads to significant overall reduction of visible colonies and high genome editing rates also when used at 21°C.

### Summary

The results obtained in Example 5.2 demonstrate that the BEC10 nuclease form the BEC family nucleases shows a significant overall colony reduction and a strong genome editing efficiency (65 %) when used at 21°C.

In contrast to this, the overall colony reduction and editing efficiency of the SuCms1 nuclease decreased significantly at 21°C down to 0.3 % which is just slightly above the editing efficiency of the negative control (0.2 %) and not suitable to work as a genome editing tool.

Furthermore, like already shown at 30°C, SeqID63 does not show any nuclease activity at all.

### 5.3. Experimental Setup (E. coli 37°C)

To evaluate the nuclease activity of the BEC family nucleases in comparison towards their next neighbor sequences at 37°C, an *E. coli* assay system was used because of its ideal growth conditions at 37°C.

To visualize the activity and efficiency of the nucleases a so-called depletion assays was carried out, wherein the survival rate of the *E. coli* cells after the nuclease targeting is monitored in comparison to a negative control (lower survival rate means better nuclease activity). As *E. coli* cells are not able to perform non homologues end joining (NHEJ) the targeting of the DNA using a CRISPR nuclease leads to cell death. Additionally, the essential rpoB gen was targeted in this experimental approach and *E*. *coli* cells are not able to survive the knockout of this gene.

For this experimental approach, the CRISPR/BEC10_Coli (SEQ ID NO: 34), CRISPR/SuCms1_Coli (SEQ ID NO: 35) or CRISPR/SeqID63_Coli (SEQ ID NO: 36) vector system was used to target the rpoB gene in *E. coli* to demonstrate the editing efficiency of the BEC family nucleases at high temperatures (37°C) in direct comparison to their next neighbor sequences SuCms1 (Begemann et al. (2017), bioRxiv) and SeqID63 (WO2019/030695).

In parallel, negative control experiments using the CRISPR/BEC10_Coli, CRISPR/SuCms1_Coli or CRISPR/SeqID63_E. coli expression constructs lacking a spacer sequence targeting the rpoB gene were performed to demonstrate the dependency of the Cas proteins to be guided to the target DNA region by a specific spacer.

After transformation and 48 h incubation at 37°C the culture plates were analyzed by counting the number of grown colonies.

### 5.4 Results

The results are summarized in Table 4 and exemplary plates are shown in Figure 5.

All experiments were carried out in 5 biological replicates and the results obtained from these replicates were combined to visualize the genome editing efficacy of BEC10 in comparison to SuCms1 and SeqID63.

**Table 4: Summary of the results of 5 experiments (plates) for each experimental setup using the E. coli depletion assay by targeting of the rpoB gene for BEC10, SuCms1 and SeqID63 (cumulated colony numbers) at 37°C**

| | **BEC10** | **SuCms** | **SeqID63** |
|---|---|---|---|
| **Negative Control** | 4963 | 4905 | 5002 |
| **Active Nuclease** | 130 | 1365 | 5025 |
| **Colony Reduction (%)** | 97 | 72 | 0 |

### CRISPR/SuCms1

Cells transformed with the negative control construct showed 4905 colonies after incubation for 48 h at 37°C whereas cells transformed with the active construct (CRISPR/SuCms1_Coli) showed 1365 colonies leading to a clone reduction of 72 %.

### CRISPR/Seq ID63

Cells transformed with the negative control construct showed 5002 colonies after incubation for 48 h at 37°C whereas cells transformed with the active construct (CRISPR/SeqID63_Coli) showed 5025 colonies leading to a clone reduction of 0 % demonstrating that SeqID63 does not show any nuclease activity in this experimental approach.

### CRISPR/BEC10

Cells transformed with the negative control construct showed 4963 colonies after incubation for 48 h at 37°C whereas cells transformed with the active construct (CRISPR/BEC10_Coli) showed 130 colonies leading to a clone reduction of 97 %.

### Summary

The results obtained in Example 5.4 demonstrate that the BEC10 nuclease shows a significant overall colony reduction (97 %) at 37°C when using the *E*. *coli*-based depletion assay, thereby proving the very high activity of the BEC10 nuclease at higher temperatures. In contrast to this, the SuCms1 nuclease showed a significantly lower decrease of the colonies (72 %) indicating the superior activity of BEC type nucleases in comparison to SuCms1 at 37°C.

Furthermore, SeqID63 does not show any nuclease activity at all with 0 % colony reduction in comparison to the negative control.

### Example 6 - Discussion of the results from Examples 3 - 5

Taken together, the results of Examples 3 - 5 show that the sequences of the newly identified and developed BEC family nucleases (BEC85, BEC67 and BEC10) which have sequence identities of ≈ 95 % to each other have comparable genome editing efficiencies based on a novel molecular genome editing mechanism when compared to Cas9 (Example 3). Furthermore, the results of Example 4 demonstrate that genome editing using the BEC family type nucleases is leading to significantly higher clone reduction numbers and significantly superior editing ratios in comparison to their next neighbor sequences SuCms1 and SeqID63 corroborating the general superiority of the BEC type nucleases for genome editing.

Most of the organisms of interest used in biotechnological, agricultural and pharmaceutical research applications are cultivated in temperatures ranging from 21°C- 37°C (e.g. various plants and plant cells ≈ 21°C, various yeast and fungal cells ≈ 30°C, various prokaryotic organisms and mammalian cell lines ≈ 37°C). Therefore, a universally applicable CRISPR system needs to show strong activity and genome editing efficiency when used in this range of temperatures. To evaluate the temperature depending activity of our newly discovered and developed BEC type nucleases experiments using the BEC10 nuclease were carried out in *S*. *cerevisiae* (21°C) and *E. coli* (37°C) (Example 5) and compared to results obtained using the next neighbor sequences SuCms1 and SeqID63. The results obtained in these experiments prove the strong activity of BEC10 at all tested temperature levels with a superior editing efficiency and colony reduction rate compared to the next neighbor sequences SuCms1 (Begemann et al. (2017), bioRxiv) and SeqID63 (WO 2019/030695). In addition to that, the editing efficiency of the SuCms1 nuclease significantly decreases at 21°C to levels comparable to the negative control (0.3 %) whereas the BEC10 editing efficiency remained at a high level (65 %) even at cooler temperatures.

## Claims

1. A nucleic acid molecule encoding an RNA-guided DNA endonuclease, which is
(a) a nucleic acid molecule encoding the RNA-guided DNA endonuclease comprising or consisting of the amino acid sequence of SEQ ID NO: 29, 1 or 3;
(b) a nucleic acid molecule comprising or consisting of the nucleotide sequence of SEQ ID NO: 30, 2 or 4;
(c) a nucleic acid molecule encoding a RNA-guided DNA endonuclease the amino acid sequence of which is at least 95 % identical to the amino acid sequence of (a);
(d) a nucleic acid molecule encoding a RNA-guided DNA endonuclease and comprising or consisting of a nucleotide sequence which is at least 95 % identical to the nucleotide sequence of (b); or
(e) the nucleic acid molecule of any one of (a) to (d) wherein T is replaced by U.

2. The nucleic acid molecule of claim 1, wherein the nucleic acid molecule is operably linked to a promoter that is native or heterologous to the nucleic acid molecule.

3. The nucleic acid molecule of claim 1 or 2, wherein said nucleic acid molecule is codon-optimized for expression in a eukaryotic cell.

4. The nucleic acid molecule of claim 3, wherein the eukaryotic cell is a plant cell or an animal cell.

5. A vector encoding the nucleic acid molecule of any one of claims 1 to 4.

6. A host cell comprising the nucleic acid molecule of any one of claims 1 to 4 or being transformed, transduced or transfected with the vector of claim 5.

7. The host cell of claim 6, wherein the host cell is a eukaryotic cell or a prokaryotic cell.

8. The host cell of claim 6, wherein the eukaryotic cell is a plant cell or an animal cell.

9. A plant, seed or a part of a plant, said part of a plant not being a single plant cell comprising the nucleic acid molecule of any one of claims 1 to 4 or being transformed, transduced or transfected with the vector of claim 5.

10. A method of producing an RNA-guided DNA endonuclease comprising culturing the host cell of any one of claims 6 to 8 and isolating the RNA-guided DNA endonuclease produced.

11. An RNA-guided DNA endonuclease encoded by the nucleic acid molecule of any one of claims 1 to 4.

12. A composition comprising the nucleic acid molecule of any one of claims 1 to 4, the vector of claim 5, the host cell of any one of claims 6 to 8, the plant, seed, part of a plant of claim 9 the RNA-guided DNA endonuclease of claim 11 or a combination thereof.

13. The composition of claim 12, wherein the composition is a pharmaceutical composition.

14. The nucleic acid molecule of any one of claims 1 to 4, the vector of claim 5, the host cell of any one of claims 6 to 8, the plant, seed, part of a plant of claim 9, the RNA-guided DNA endonuclease of claim 11 or a combination thereof for use in the treatment of a disease in a subject by modifying a nucleotide sequence at a target site in the genome of the subject.

15. An *in vitro* method of modifying a nucleotide sequence at a target site in the genome of a cell comprising introducing into said cell
(i) a DNA-targeting RNA or a DNA polynucleotide encoding a DNA-targeting RNA, wherein the DNA-targeting RNA comprises:
(a) a first segment comprising a nucleotide sequence that is complementary to a sequence in the target DNA; and
(b) a second segment that interacts with the RNA-guided DNA endonuclease of claim 11; and
(ii) the RNA-guided DNA endonuclease of claim 11, or the nucleic acid molecule encoding an RNA-guided DNA endonuclease of any one of claims 1 to 4, or the vector of claim 5, wherein the RNA-guided DNA endonuclease comprises:
(a) an RNA-binding portion that interacts with the DNA-targeting RNA; and
(b) an activity portion that exhibits site-directed enzymatic activity.

16. The method of claim 15, wherein in case the RNA-guided DNA endonuclease and the DNA-targeting RNA are directly introduced into the cell they are introduced in the form of a ribonucleoprotein complex (RNP).

17. A diagnostic composition comprising the RNA-guided DNA endonuclease of claim 11 and a single-stranded DNA strand being attached to a marker, so that when the RNA-guided DNA endonuclease polypeptide cuts the single-stranded DNA, it activates the reporter, causing it to fluoresce or change color.

## Patentansprüche

1. Nukleinsäuremolekül, das eine RNA-angeleitete DNA-Endonuklease kodiert, die
(a) ein Nukleinsäuremolekül ist, das die RNA-angeleitete DNA-Endonuklease kodiert, die die Aminosäuresequenz der SEQ ID NO: 29, 1 oder 3 umfasst oder daraus besteht,
(b) ein Nukleinsäuremolekül ist, das die Nukleotidsequenz der SEQ ID NO: 30, 2 oder 4 umfasst oder daraus besteht,
(c) ein Nukleinsäuremolekül ist, das eine RNA-angeleitete DNA-Endonuklease kodiert, deren Aminosäuresequenz zu mindestens 95% identisch zur Aminosäuresequenz nach (a) ist;
(d) ein Nukleinsäuremolekül ist, das eine RNA-angeleitete DNA-Endonuklease kodiert und eine Nukleinsäuresequenz umfasst oder daraus besteht, die zu mindestens 95% identisch zur Nukleinsäuresequenz nach (b) ist;
(e) das Nukleinsäuremolekül nach einem von (a) bis (d) ist, wobei T durch U ausgetauscht ist.

2. Nukleinsäuremolekül nach Anspruch 1, wobei das Nukleinsäuremolekül funktionell mit einem Promoter verknüpft ist, der nativ oder heterolog zum Nukleinsäuremolekül ist.

3. Nukleinsäuremolekül nach Anspruch 1 oder 2, wobei das Nukleinsäuremolekül für die Expression in einer eukaryotischen Zelle Kodon-optimiert ist.

4. Nukleinsäuremolekül nach Anspruch 3, wobei die eukaryotische Zelle eine Pflanzenzelle oder eine Tierzelle ist.

5. Vektor, der das Nukleinsäuremolekül nach einem der Ansprüche 1 bis 4 kodiert.

6. Wirtszelle, die das Nukleinsäuremolekül nach einem der Ansprüche 1 bis 4 umfasst oder mit dem Vektor nach Anspruch 5 transformiert, transduziert oder transfiziert ist.

7. Wirtszelle nach Anspruch 6, wobei die Wirtszelle eine eukaryotische Zelle oder eine prokaryotische Zelle ist.

8. Wirtszelle nach Anspruch 6, wobei die eukaryotische Zelle eine Pflanzenzelle oder eine Tierzelle ist.

9. Pflanze, Saatgut oder Teil einer Pflanze, der keine einzelne Pflanzenzelle ist, die/das/der das Nukleinsäuremolekül nach einem der Ansprüche 1 bis 4 umfasst oder mit dem Vektor nach Anspruch 5 transformiert, transduziert oder transfiziert ist.

10. Verfahren zur Herstellung einer RNA-angeleiteten DNA-Endonuklease, umfassend die Züchtung einer Wirtszelle nach einem der Ansprüche 6 bis 8 und die Isolierung der hergestellten RNA-angeleiteten DNA-Endonuklease.

11. RNA-angeleitete DNA-Endonuklease, die durch das Nukleinsäuremolekül nach einem der Ansprüche 1 bis 4 kodiert ist.

12. Zusammensetzung, umfassend das Nukleinsäuremolekül nach einem der Ansprüche 1 bis 4, den Vektor nach Anspruch 5, die Wirtszelle nach einem der Ansprüche 6 bis 8, die Pflanze, das Saatgut oder den Teil einer Pflanze nach Anspruch 9, die RNA-angeleitete DNA-Endonuklease nach Anspruch 11 oder eine Kombination davon.

13. Zusammensetzung nach Anspruch 12, wobei die Zusammensetzung eine pharmazeutische Zusammensetzung ist.

14. Nukleinsäuremolekül nach einem der Ansprüche 1 bis 4, Vektor nach Anspruch 5, Wirtszelle nach einem der Ansprüche 6 bis 8, Pflanze, Saatgut oder Teil einer Pflanze nach Anspruch 9, RNA-angeleitete DNA-Endonuklease nach Anspruch 11 oder eine Kombination davon zur Verwendung in der Behandlung einer Krankheit in einem Individuum durch das Modifizieren einer Nukleotidsequenz an einer Zielstelle in dem Genom des Individuums.

15. *In vitro* Verfahren zum Modifizieren einer Nukleinsäuresequenz an einer Zielstelle in dem Genom in einer Zelle, umfassend das Einführen in die Zelle:
(i) eines auf DNA gerichteten RNA- oder eines DNA-Polynukleotids, das ein auf DNA gerichtetes RNA-Polynukleotid kodiert, wobei die auf DNA gerichtete RNA umfasst:
(a) ein erstes Segment, das eine Nukleinsäuresequenz umfasst, die komplementär zu einer Sequenz in der Ziel-DNA ist; und
(b) ein zweites Segment, das mit der RNA-angeleiteten DNA-Endonuklease nach Anspruch 11 interagiert;
(ii) die RNA-angeleitete DNA-Endonuklease nach Anspruch 11 oder das Nukleinsäuremolekül, das eine RNA-angeleitete DNA-Endonuklease nach einem der Ansprüche 1 bis 4 kodiert, oder den Vektor nach Anspruch 5, wobei die RNA-angeleitete DNA-Endonuklease umfasst:
(a) einen RNA bindenden Teil, der mit der auf DNA gerichtetes RNA interagiert; und
(b) einen Aktivitätsteil, der stellenspezifische enzymatische Aktivität hat.

16. Verfahren nach Anspruch 15, wobei im Fall, dass die RNA-angeleitete DNA-Endonuklease und die auf DNA gerichtete RNA direkt in die Zelle eingebracht werden, diese in Form eines Ribonukleoproteinkomplexes (RNP) eingebracht werden.

17. Diagnostische Zusammensetzung, umfassend die RNA-angeleitete DNA-Endonuklease nach Anspruch 11 und einen einzelsträngigen DNA-Strang, der an einen Marker gebunden ist, so dass wenn das RNA-angeleitete DNA-Endonuklease-Polypeptid den einzelsträngigen DNA-Strang schneidet, es den Reporter aktiviert, was eine Fluoreszenz-oder Farbänderung zur Folge hat.

## Revendications

1. Molécule d'acide nucléique codant une endonucléase d'ADN guidée par un ARN, qui est
(a) une molécule d'acide nucléique codant l'endonucléase d'ADN guidée par un ARN comprenant ou consistant en la séquence d'acides aminés de la SEQ ID NO: 29, 1 ou 3;
(b) une molécule d'acide nucléique comprenant ou consistant en la séquence de nucléotides de la SEQ ID NO : 30, 2 ou 4;
(c) une molécule d'acide nucléique codant une endonucléase d'ADN guidée par un ARN dont la séquence d'acides aminés est identique à au moins 95 % à la séquence d'acides aminés de (a);
(d) une molécule d'acide nucléique codant une endonucléase d'ADN guidée par un ARN et comprenant ou consistant en une séquence de nucléotides qui est identique à au moins 95 % à la séquence de nucléotides de (b); ou
(e) la molécule d'acide nucléique de l'un quelconque de (a) à (d) dans laquelle T est remplacé par U.

2. Molécule d'acide nucléique selon la revendication 1, laquelle molécule d'acide nucléique est liée de manière fonctionnelle à un promoteur qui est natif ou hétérologue de la molécule d'acide nucléique.

3. Molécule d'acide nucléique selon la revendication 1 ou 2, laquelle molécule d'acide nucléique est optimisée au niveau du codon pour une expression dans une cellule eucaryote.

4. Molécule d'acide nucléique selon la revendication 3, dans laquelle la cellule eucaryote est une cellule végétale ou une cellule animale.

5. Vecteur codant la molécule d'acide nucléique selon l'une quelconque des revendications 1 à 4.

6. Cellule hôte comprenant la molécule d'acide nucléique selon l'une quelconque des revendications 1 à 4, ou transformée, transduite ou transfectée par le vecteur de la revendication 5.

7. Cellule hôte selon la revendication 6, laquelle cellule hôte est une cellule eucaryote ou une cellule procaryote.

8. Cellule hôte selon la revendication 6, dans laquelle la cellule eucaryote est une cellule végétale ou une cellule animale.

9. Plante, graine ou partie d'une plante, ladite partie d'une plante n'étant pas une cellule végétale isolée, comprenant la molécule d'acide nucléique selon l'une quelconque des revendications 1 à 4, ou transformée, transduite ou transfectée par le vecteur de la revendication 5.

10. Méthode de production d'une endonucléase d'ADN guidée par un ARN, comprenant la culture de la cellule hôte selon l'une quelconque des revendications 6 à 8 et l'isolation de l'endonucléase d'ADN guidée par un ARN produite.

11. Endonucléase d'ADN guidée par un ARN codée par la molécule d'acide nucléique selon l'une quelconque des revendications 1 à 4.

12. Composition comprenant la molécule d'acide nucléique selon l'une quelconque des revendications 1 à 4, le vecteur de la revendication 5, la cellule hôte selon l'une quelconque des revendications 6 à 8, la plante, graine, partie d'une plante de la revendication 9, l'endonucléase d'ADN guidée par un ARN de la revendication 11, ou une de leurs combinaisons.

13. Composition selon la revendication 12, laquelle composition est une composition pharmaceutique.

14. Molécule d'acide nucléique selon l'une quelconque des revendications 1 à 4, vecteur selon la revendication 5, cellule hôte selon l'une quelconque des revendications 6 à 8, plante, graine, partie d'une plante selon la revendication 9, endonucléase d'ADN guidé par un ARN selon la revendication 11, ou une combinaison de celles-ci, pour son utilisation dans le traitement d'une maladie chez un sujet par modification d'une séquence de nucléotides au niveau d'un site cible dans le génome du sujet.

15. Méthode *in vitro* de modification d'une séquence de nucléotides au niveau d'un site cible dans le génome d'une cellule, comprenant l'introduction dans ladite cellule
(i) d'un ARN ciblant un ADN ou d'un polynucléotide d'ADN codant un ARN ciblant un ADN, parmi lesquels l'ARN cible un ADN comprend:
(a) un premier segment comprenant une séquence de nucléotides qui est complémentaire d'une séquence dans l'ADN cible; et
(b) un deuxième segment qui interagit avec l'endonucléase d'ADN guidée par un ARN de la revendication 11; et
(ii) d'une endonucléase d'ADN guidée par un ARN de la revendication 11, ou de la molécule d'acide nucléique codant une endonucléase d'ADN guidée par un ARN selon l'une quelconque des revendications 1 à 4, ou le vecteur de la revendication 5, laquelle endonucléase d'ADN guidée par un ARN comprend:
(a) une portion se liant à un ARN qui interagit avec l'ARN ciblant un ADN; et
(b) une portion d'activité qui présente une activité enzymatique dirigée.

16. Méthode selon la revendication 15, dans laquelle, dans le cas où l'endonucléase d'ADN guidée par un ARN et l'ARN ciblant un ADN sont directement introduits dans la cellule, ils sont introduits sont la forme d'un complexe ribonucléoprotéique (RNP).

17. Composition diagnostique comprenant l'endonucléase d'ADN guidée par un ARN de la revendication 11 et un brin d'ADN simple brin attaché à un marqueur, de façon que, lorsque le polypeptide d'endonucléase d'ADN guidée par un ARN coupe l'ADN simple brin, il active le rapporteur, le faisant entrer en fluorescence ou changer de couleur.
